# EUROPEAN PATENT APPLICATION

(11) **EP 0 550 025 A1**
(43) Date of publication of application: **07.07.1993**
(21) Application number: 92121959.8
(22) Date of filing: 23.12.1992
(51) Int. Cl.: C07D 209/54, C07D 221/20

(54) **Azaspiroheptanes and octanes and processes for their production**

(30) Priority: 31.12.1991 KR 25885
(71) Applicant: KOREA RESEARCH INSTITUTE OF CHEMICAL TECHNOLOGY, Daejeon, 305-606 (KR)
(72) Inventor: Kim, Wan Joo, Daejeon 305-606 (KR); Park, Myung Hwan, Daejeon 305-606 (KR); Ha, Jae Du, Daejeon 305-606 (KR); Baik, Kyong Up, Daejeon 302-181 (KR)
(74) Representative: Fischer, Hans-Jürgen, Dr.

(57) **Abstract**

The present invention relates to certain novel azasprio derivatives of following formula(I), which are especially useful as intermediates for the preparation of quinolone carboxylic acid derivatives posssessed with a broad spectrum of potent anti-bacterial activities; and the novel processes for preparing these intermediates. 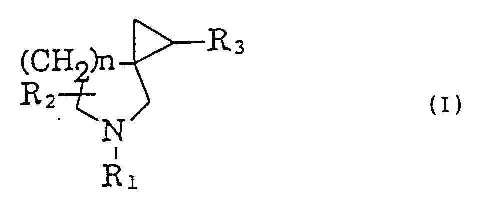
wherein:
- R₁ is: a hydrogen atom, an optionally substituted C₁₋₂₀ alkyl, optionally substituted benzyl, or nitrogen protecting group;
- R₂ is: a hydrogen atom or a methyl group which is preferably substituted at the 4- or 6-position carbon;
- R₃ is: -CO₂H, -CON₃, -NCO, -CONHR₁₀ wherein R₁₀ is a hydrogen atom, a benzyl, C₁₋₈ alkyl, or C₁₋₅ alkoxycarbonyl group, or -(CH₂)mY wherein m is 0 or 1 and Y is a hydroxyl, C₂₋₅ acyloxy, mesyloxy, p-toluensulfonyloxy or amino group which is optionally substituted with one or two C₁₋₄ alkyl radicals, with one C₁₋₄ alkanoyl radical, with one benzoyl radical or with one nitrogen protecting radical metabolizable in vivo; and the carbon atom to which R₃ is attached is a chiral carbon atom which has the stereochemical configuration of (1,3)-R,S, (1,3)-R,R, (1,3)-S,S or (1,3)-S, R; and
- n is: 1 or 2.

## Description

### Field of Invention

The present invention relates to certain novel azaspiro derivatives, which are especially useful as intermediates for the preparation of quinolone carboxylic acid derivatives possessed with a broad spectrum of potent anti-bacterial activities, and to novel processes for preparing these intermediates.

### Description of the Prior Art

Quinolones such as enoxacin, norfloxacin, ofloxacin, ciprofloxacin, tosulfloxacin and the like have been developed and commercially available for sometime. However, most of these prior art materials are known to have various side effects on the central nervous system or cardiovascular system; and resistant microorganisms against these drugs, e.g., clinically important methicillin resistant Staphylococcus aureus(MRSA), have been found to exist.

Therefore, needs have existed for the development of new antibiotics which possess a broad spectrum of potent anti-bacterial activities against both Gram-positive and Gram-negative bacteria and are effective against quinolone-resistant microorganisms including said MRSA, and without the problem of causing side effects.

Recently, the present inventors have discovered that certain quinolone carboxylic acid derivatives having certain substituents at 7-position of the quinolone nucleus, which can be represented by the formula(A) as defined below, satisfy said requirements: 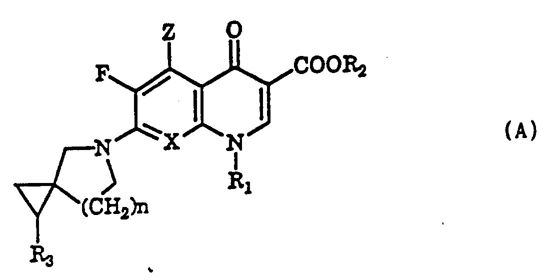
wherein:
- X is: a nitrogen atom or a CH, C-halogen or C-methoxy group;
- R₁ is: an optionally substituted C₁₋₇ alkyl group, a C₃₋₇ cycloalkyl, optionally substituted alkenyl or optionally substituted aryl group or a divalent group of -CH₂CH₂*CH(CH₃)-, -OCH₂*CH(CH₃)- or -SCH₂*CH(CH₃)- which forms a ring together with the nitrogen atom to which R₁ is attached and with X wherein X is CH;
- R₂ is: a hydrogen atom, a carboxy protecting group or a pharmaceutically acceptable metal or organic cation;
- R₃ is: -CO₂H or -(CH₂)ₘ-Y, wherein m is 0 or 1 and Y is a hydroxy group, a C₁₋₄ alkoxy group, a C₁₋₄ alkoxycarbonyl group(wherein m is 0) or an amino group optionally substituted with one or two C₁₋₄ alkyl radicals, with one C₁₋₄ alkanoyl radical or with one nitrogen protecting radical metabolizable in vivo; and the carbon atom to which R₃ is attached is a chiral carbon atom having the stereochemical configuration of (R), (S) or a mixture thereof;
- Z is: a hydrogen, chlorine or fluorine atom, a hydroxy, methyl or optionally substituted amino group; and
- n is: 1 or 2.

The compounds of formula(A) can be prepared in accordance with the methods described in Korean Patent Application No. 91-25884 entitled "Novel Quinolone Carboxylic Acid Derivatives and Processes for Preparing Same" (corresponding to WO-A.......).

### Summary of the Invention

Unexpectedly, the present inventors have discovered that novel compounds of formula(I) described below can be effectively employed as the starting material to produce such potent quinolone carboxylic acid derivatives of formula(A) with anti-bacterial activities.

Accordingly, the present invention primarily pertains to said novel azaspiro derivatives or their pharmaceutically acceptable salts which are useful as intermediates for the preparation of said quinolone carboxylic acid derivatives, and novel processes for preparing such intermediates.

### Detailed Description of the Invention

The novel azaspiro derivatives of the present invention may be represented by the following formula(I): 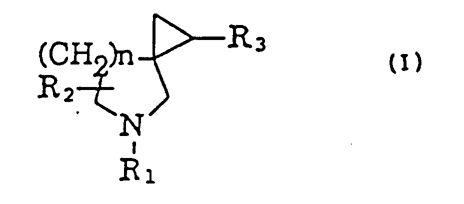
wherein:
- R₁ is: a hydrogen atom, an optionally substituted C₁₋₂₀ alkyl, optionally substituted benzyl, or nitrogen protecting group;
- R₂ is: a hydrogen atom or a methyl group which is preferably substituted at the 4- or 6-position carbon;
- R₃ is: -CO₂H, -CON₃, -NCO, -CONHR₁₀ wherein R₁₀ is a hydrogen atom, a benzyl, C₁₋₈ alkyl, or C₁₋₅ alkoxycarbonyl group, or -(CH₂)mY wherein m is 0 or 1 and Y is a hydroxyl C₂₋₅ acyloxy, mesyloxy, p-toluensulfonyloxy or amino group which is optionally substituted with one or two C₁₋₄ alkyl radicals, with one C₁₋₄ alkanoyl radical, with one benzyl radical or with one nitrogen protecting radical metabolizable in vivo; and the carbon atom to which R₃ is attached is a chiral carbon atom which has the stereochemical configuration of (1,3)-R,S, (1,3)-R,R, (1,3)-S,S or (1,3)-S,R; and
- n is: 1 or 2.

With respect to the definition for R₁ of the compounds of formula(I), in the case that R₁ is an optionally substituted C₁₋₂₀ alkyl group: preferred is a methyl, ethyl, propyl, isopropyl, butyl, t-butyl, decanoyl, octyl or dodecanoyl group;
in the case that R₁ is an optionally substituted benzyl group: preferred is a benzyl, 4-nitrobenzyl, 4-methyoxybenzyl, 4-fluorobenzyl or 2,4-difluorobenzyl group;and
in the case that R₁ is a nitrogen protecting group: preferred is a formyl, optionally substituted C₁₋₅ alkylcarbonyl, optionally substituted benzoyl, optionally substituted C₁₋₅ alkoxycarbonyl, optionally substituted benzyloxycarbonyl, optionally substituted C₁₋₅ alkylsulfonyl or optionally substituted benzensulfonyl group.

With respect to the definition for R₃ of the compounds of formula(I), in the case that R₃ is an amino group substituted with a nitrogen-protecting radical metabolizable in vivo: preferred nitrogen-protecting radical is a formyl, C₁₋₄ alkoxycarbonyl, C₁₋₄ alkylcarbonyl methyl, 2-or 3-oxoalkyl, 4-methylene-5-methyl-1,3-dioxorene-2-one or RCH(NH₂)CO group derived from an amino acid wherein R is a hydrogen atom, an optionally substituted C₁₋₄ alkyl group or a phenyl group.

Among the compounds of the present invention, preferred are:
5-benzyloxycarbonyl-1-ethoxycarbonyl-5-azaspiro[2,4]heptane; 5-benzyloxycarbonyl-5-azaspiro[2,4]heptane-1-carboxylic acid; 5-benzyloxycarbonyl-1-azidocarbonyl-5-azaspiro[2,4]heptane; 5-benzyloxycarbonyl-1-isocyanato-5-azaspiro[2,4]heptane; 1-amino-5-azaspiro[2,4]heptane hydrochloride; 1-amino-5-azaspiro[2,4]heptane, 5-benzyl-1-ethoxycarbonyl-5-azaspiro[2,4]heptane; bis-(1,5-ethoxycarbonyl)-5-azaspiro[2,4]heptane; 5-ethoxycarbonyl-5-azaspiro[2,4] heptane-1-carboxylic acid; 5-(t-butoxycarbonyl)-1-ethoxycarbonyl-5-azaspiro[2,4]heptane; 5-t-butoxycarbonyl-1-t-butoxycarbonylamino-5-azaspiro[2,4]heptane; 1-t-butoxycarbonyl-methylamino-5-t-butoxycarbonyl-5-azaspiro[2,4]heptane; 1-methylamino-5-azaspiro[2,4]heptane; 1-methylamino-5-azaspiro[2,4]heptane hydrochloride; 5-benzyl-1-(ethoxycarbonyl)-5-azaspiro[2,5]octane; 5-benzyloxycarbonyl-1-ethyloxycarbonyl-5-azaspiro[2,5]octane; 5-benzyloxycarbonyl-5-azaspiro[2,5]octane-1-carboxylic acid; 1-amino-5-azaspiro[2,5]octane; 1-amino-5-azaspiro[2,5]octane hydrochloride; 5-benzyl-1-benzylamido-5-azaspiro[2,4]heptane; 5-benzyl-1-benzylaminomethyl-5-azaspiro [2,4]heptane; 1-aminomethyl-5-azaspiro[2,4]heptane; 1-aminomethyl-5-azaspiro[2,4]heptane formate; 1-hydroxymethyl-5-benzyl-5-azaspiro [2,4]heptane; 1-hydroxymethyl-5-azaspiro[2,4]heptane; 1-hydroxymethyl-5-azaspiro[2,4]heptane formate; 1-amino-5-ethoxycarbonyl-5-azaspiro[2,4]heptane; 1-hydroxy-5-azaspiro[2,4]heptane hydrochloride; 1-acetoxymethyl-5-t-butoxycarbonyl-5-azasprio[2,4]heptane; 5-benzyl-1-ethoxycarbonyl-4-methyl-5-azaspiro[2,4]heptane; 5-benzyl-1-ethoxycarbonyl-6-methyl-5-azaspiro[2,4]heptane; 5-ethoxycarbonyl-1-ethoxycarbonyl-4-methyl-5-azaspiro[2,4]heptane; 5-ethoxycarbonyl-1-ethoxycarbonyl-6-methyl-5-azaspiro[2,4]heptane; 5-ethoxycarbonyl-4-methyl-5-azaspiro[2,4]heptane-1-carboxylic acid; 5-ethoxycarbonyl-6-methyl-5-azaspiro[2,4]heptane-1-carboxylic acid; 5-ethoxycarbonyl-6-methyl-1-azidocarbonyl-5-azaspiro[2,4]heptane; 5-ethoxycarbonyl-4-methyl-1-azidocarbonyl-5-azaspiro[2,4]heptane; 5-ethoxycarbonyl-1-azidocarbonyl-5-azaspiro[2,4]heptane; 5-ethoxycarbonyl-1-isocyanato-5-azaspiro[2,4]heptane; 5-ethoxycarbonyl-1-isocyanato-4-methyl-5-azaspiro[2,4]heptane; 5-ethoxycarbonyl-1-isocyanato-6-methyl-5-azaspiro[2,4]heptane; 1-amino-4-methyl-5-azaspiro[2,4]heptane; 1-amino-4-methyl-5-azaspiro[2,4]heptane hydrochloride; 1-amino-6-methyl-5-azaspiro[2,4]heptane; 1-amino-6-methyl-5-azaspiro[2,4] heptane hydrochloride; N-[1-(R)-phenylethyl]-5-(t-butoxycarbonyl)-5-azaspiro[2,4]heptane-1-carboxamide; (1S,3S)-N-[1-(R)-phenylethyl]-5-(t-butoxycarbonyl)-5-azaspiro[2,4]heptane-1-carboxamide; (1R,3R)-N-[1-(R)-phenylethyl]-5-(t-butoxycarbonyl)-5-azaspiro[2,4]heptane-1-carboxamide; (1R,3S)-N-[1-(R)-phenylethyl]-5-(t-butoxycarbonyl)-5-azaspiro[2,4]heptane-1-carboxamide; (1S,3R)-N-[1-(R)-phenylethyl]-5-(t-butoxycarbonyl)-5-azaspiro[2,4]heptane-1-carboxamide; (1R,3R)-azaspiro[2,4]heptane-1-carboxylic acid; (1S,3S)-5-azaspiro[2,4]heptane-1-carboxylic acid; (1S,3R)-5-azaspiro[2,4]heptane-1-carboxylic acid; (1R,3S)-5-azaspiro[2,4]heptane-1-carboxylic acid; (1S,3S)-5-(t-butoxycarbonyl)-5-azaspiro[2,4]heptane-1-carboxylic acid; (1R,3R)-5-(t-butoxycarbonyl)-5-azaspiro[2,4]heptane-1-carboxylic acid; (1S,3R)-5-(t-butoxycarbonyl)-5-azaspiro[2,4]heptane-1-carboxylic acid; (1R,3S)-5-(t-butoxycarbonyl)-5-azaspiro[2,4]heptane-1-carboxylic acid; (1S,3R)-1-amino-5-azaspiro[2,4]heptane; (1S,3R)-1-amino-5-azaspiro [2,4]heptane hydrochloride; (1S,3S)-1-amino-5-azaspiro[2,4]heptane; (1R,3R)-1-amino-5-azaspiro[2,4]heptane; (1S,3S)-1-amino-5-azaspiro [2,4]heptane hydrochloride; (1R,3R)-1-amino-5-azaspiro[2,4]heptane hydrochloride; (1R,3S)-1-amino-5-azaspiro[2,4]heptane; (1R,3S)-1-amino-5-azaspiro[2,4]heptane hydrochloride; 1-methanesulfonyloxymethyl-5-benzyl-5-azaspiro[2,4]heptane and 5-benzyl-1-(p-toluenesulfonyloxymethyl)-5-azaspiro[2,4]heptane.

More preferred compounds of the present invention are: 1-amino-5-azaspiro[2,4]heptane, (1S,3S)-1-amino-5-azaspiro[2,4] heptane, (1S,3R)-1-amino-5-azaspiro[2,4]heptane, (1R,3S)-1-amino-5-azaspiro[2,4]heptane, (1R,3R)-1-amino-5-azaspiro[2,4]heptane or 1-aminomethyl-5-azaspiro[2,4]heptane, 1-amino-5-azaspiro[2,4]octane and 1-methylamino-5-azaspiro[2,4]heptane or their hydrochloride salt.

The azaspiro compounds of the present invention represented by formula(I) can be prepared as follows: 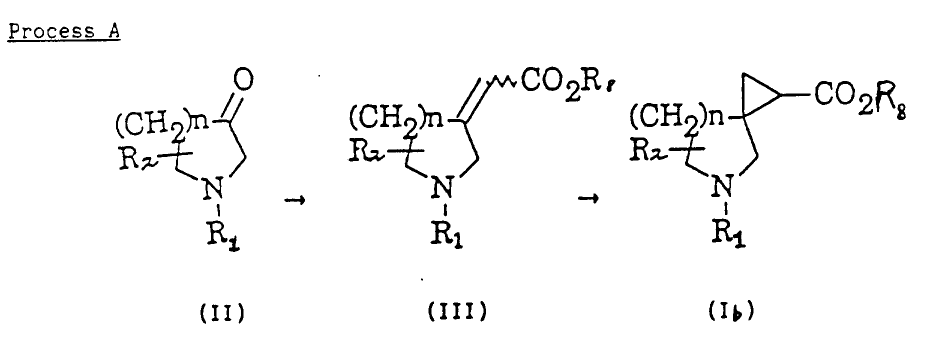
wherein:
- R₁, R₂ and n are: the same as defined in the formula(I); and
- R₈ is: a C₁₋₅ alkyl group, preferably a methyl or ethyl group.

In accordance with the above Process A, first, the compounds of formula(II) are reacted with a triethylphosphono acetate or the compounds of formula P(C₆H₅)₃CH₂CO₂R₈ · X, wherein R₈ is the same as defined previously and X is an iodine or bromine atom, in the presence of a strong base to obtain the compounds of formula (III); and, subsequently, said compounds of formula(III) are reacted with methylene diiodide or methylene dichloride, and zinc dust or titanium(IV) chloride to produce the compounds of formula(Ib).

In the first step, the preferred base used is a strong base such as sodium hydride, potassium hydride, sodium methoxide, sodium ethoxide, sodium t-butoxide, potassium t-butoxide, butyl lithium, potassium hydroxide, sodium hydroxide, potassium carbonate, sodium carbonate and the like. The above reaction in the first step can be carried out in a non-reactive solvent such as tetrahydrofuran, ethylether, benzene, toluene, dioxane, diethylsulfoxide or a mixture thereof, at a temperature between -20°C and the boiling point of the solvent used. In the second step, such a solvent as tetrahydrofuran, methylene chloride etc., may also be preferably used.

The compounds of formula(Ia) can be prepared from the compounds of formula(Ib) in accordance with either Process B or C given below: 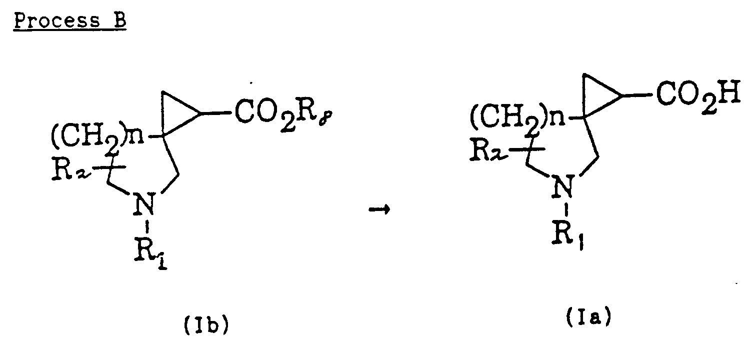
wherein:
- R₁, R₂ and n are: the same as defined previously; and
- R₈ is: a C₁₋₅ alkyl group.

In accordance with Process B, the compounds of formula(Ia) can be prepared by hydrolyzing the compounds of formula(Ib) in an aqueous 0.5% to 10% sodium hydroxide or potassium hydroxide dissolved in an alcohol, followed by acidifying the resultant with an conventional acid. Preferred alcohol used in the reaction is a methanol or ethanol. This hydrolysis can be also conducted in an alkaline solution and tetrahydrofuran. Such a surfactant as benzyltriethyl chloride or tetrabutylammonium bromide may also be used. 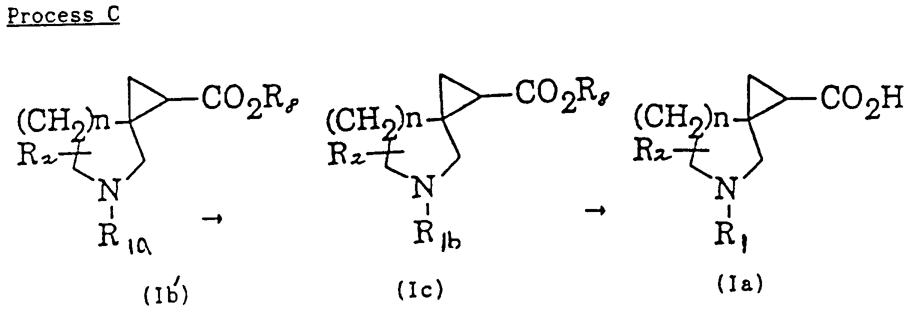
wherein:
- R₂ and n are: the same as defined in the formula(I);
- R₁ₐ is: an optionally substituted benzyl group;
- R_{1b} is: an optionally substituted C₁₋₅ alkoxycarbonyl or optionally substituted benzyloxycarbonyl group; and
- R₈ is: a C₁₋₅ alkyl group.

In Process C, for the preparation of the compounds of formula(I) wherein R₁ is an optionally substituted benzyl group, the compounds of formula(Ib' ) are reacted with ethylchloroformate or benzyl chloroformate in a solvent such as benzene, dichloromethane and the like at a temperature between a room temperature and the boiling point of the solvent used to produce the compounds of formula (Ic), or the compounds of formula(Ib' ) are subjected to a reduction reaction with hydrogen in accordance with a conventional method to remove the R₁ group; and the resultant is then reacted with di-t-butyl dicarbonate, benzyl or alkylchloroformate in the presence of a conventional base to produce the compounds of formula(Ic). Subsequently, the resulting compounds of formula(Ic) are treated in the same manner as described in the above Process B to obtain the compounds of formula(Ia).

The said compounds(Ib) can be prepared by using either Process D or Process E given below: 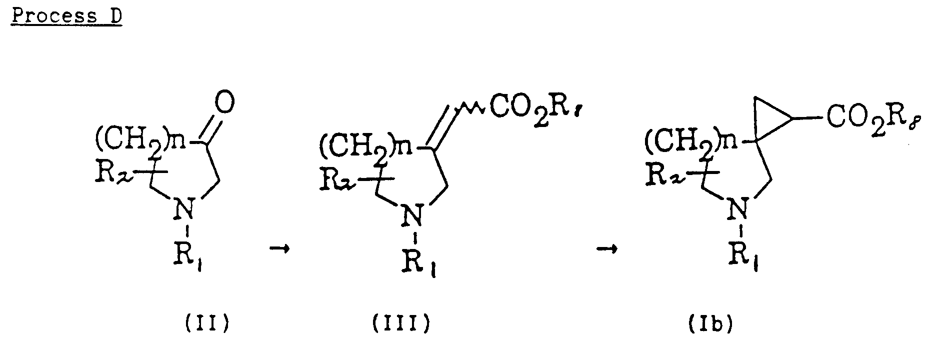
wherein:
- R₁, R₂ and n are: the same as defined in the formula(I); and
- R₈ is: a C₁₋₅ alkyl group.

In accordance with Process D, the known compounds of formula(II) are treated in the same manner as in Process A to produce the compounds of formula(III); and, thereafter, said compounds of formula(III) are reacted with trimethylsulfoxonium iodide or trimethylsulfonium iodide, preferably in the presence of a strong base such as sodium, sodium hydride, and potassium hydride in a solvent such as dimethylsulfoxide, tetrahydrofuran, dioxane or a mixture thereof at a temperature between a room temperature and 100°C to produce the compounds of formula(Ib). 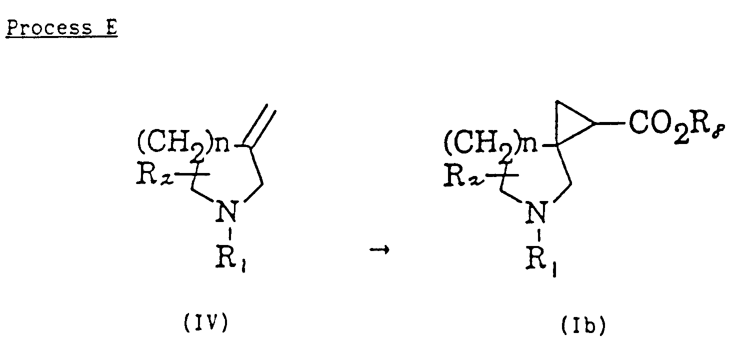
wherein:
- R₁, R₂ and n are: the same as defined in the formula(I); and
- R₈ is: a C₁₋₅ alkyl group.

In accordance with Process E, the compounds of formula(Ib) can also be prepared by reacting the compounds of formula(IV) in the presence of a catalyst such as rhodium acetate dimer, cuprous chloride and sufuric acid, etc. with ethyl diazoacetate in a solvent such as cyclohexane and the like, while heating.

The compounds of formula(I) wherein R₃ is an azidocarbonyl group, which are referred to as the compounds of formula(Id) hereinafter, can be prepared by employing Process F given below: 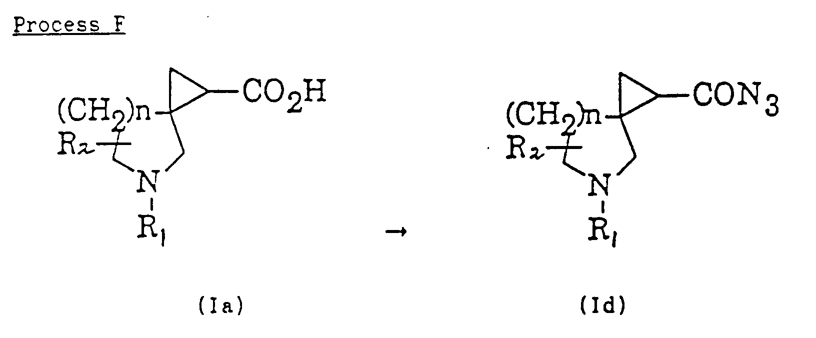
wherein:
- R₁, R₂ and n are: the same as defined in the formula(I).

In Process F, an optionally substituted C₁₋₅ alkylchloroformate is added to said compounds of formula(Ia) in the presence of a base; and the resultant is further reacted with an aqueous sodium azide solution to produce the compounds of formula(Id).

Said alkylchloroformate compounds which may be used in the above reaction is preferably methylchloroformate, ethylchloroformate or buthylchloroformate; and the base which may be used is preferably triethylamine, diisopropylethylamine, sodium hydroxide, potassium hydroxide, sodium carbonate or potassium carbonate. Preferably, this reaction may be practised in a solvent such as acetone, dichloromethane, tetrahydrofuran or a mixture thereof at a temperature ranging from -20°C to a room temperature.

The compounds of formula(I) wherein R₃ is isocyanate, which are referred to as the compounds of formula(Ie) hereinafter, can be prepared as follows: 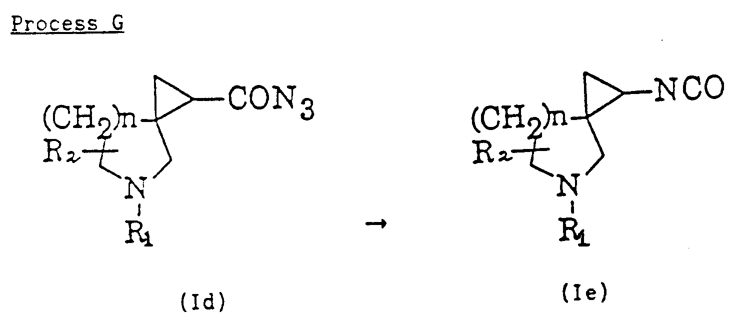
wherein:
- R₁, R₂ and n are: the same as defined above.

In accordance with Process G, the compounds of formula(Ie) can be prepared by heating the compounds of formula(Id) in a non-reactive solvent at the boiling point of the solvent used.

Preferred non-reactive solvent is toluene, xylene, benzene, or a mixture thereof.

The compounds of formula(I) wherein R₃ is an amino group, which are referred to as the compounds of formula(If) hereinafter, can be prepared from the compounds of formula(Ie) by using Process H given below: 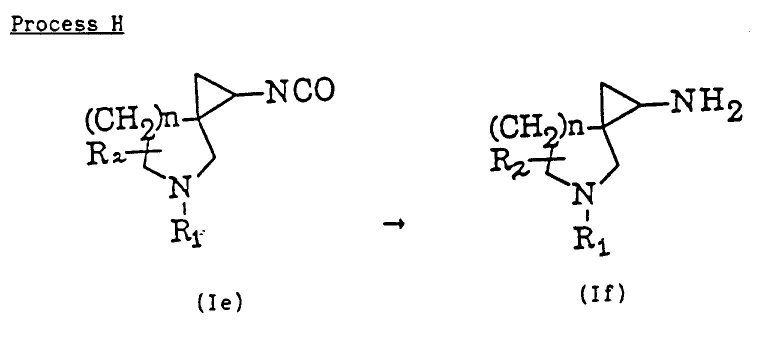
wherein:
- R₁, R₂ and n are: the same as defined in the formula(I).

In accordance with Process I, the compounds of formula(If) can be prepared by hydrolyzing the compounds of formula(Ie) in the presence of an acid, at a temperature ranging from -5°C to a room temperature or with heating under reflux, or by hydrolyzing the compounds of formula(Ie) in the presence of a alkali at a temperature ranging from a room temperature to 110°C.

The preferred acid used in Process H is 1 to 20% of aqueous hydrochloric acid, hydrobromic acid or sulfuric acid. The preferred alkali used in Process H is 1 to 10% of aqueous sodium hydroxide or potassium hydroxide.

The R₁ group in the compounds of formula(If) thus obtained can be converted to a hydrogen atom by employing a reagent such as sodium hydroxide, potassium hydroxide, 1-36% hydrochloric acid, formic acid, 1-47% hydrobromic acid, 1-30% sulfuric acid or hydrogen bromide-acetic acid. Then, the compounds of formula(If), wherein R₂ is a hydrogen atom, can be converted to the acid addition salts thereof with a conventional organic or inorganic acid such as hydrochloric, hydrobromic, sulfuric, phosphoric, formic, acetic, trifluoroacetic, propionic, lactic, citric, succinic, maleic, malonic, fumaric, tartaric, ascorbic, glutamic, methanesulfonic, benzenesulfonic or p-toluenesulfonic acid.

The compounds of formula(I) wherein R₃ is a hydroxy group, which are referred to as the compounds of formula(Ig) hereinafter, can be prepared by employing the following Process I: 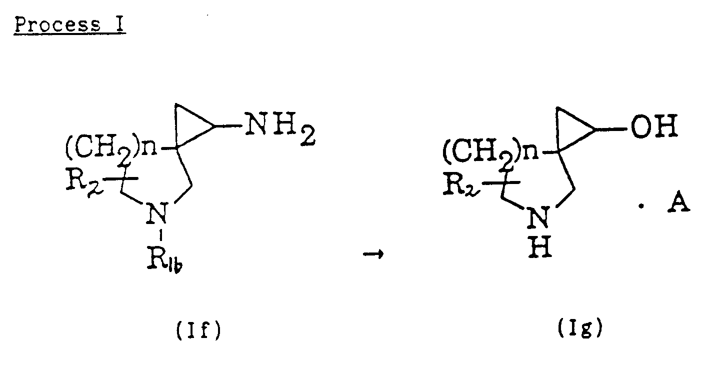
wherein:
- R₁, R₂ and n are: the same as defined in the formula(I); and
- A is: hydrochloric, sulfuric or nitric acid.

In Process I, the compouns of formula(Ig) can be produced by diazotization of the compounds of formula(If) with sodium nitrite end hydrochloric or sulfuric acid in accordance with a conventional procedure, followed by heating the reaction mixture.

The compounds of formula(I) wherein R₃ is -NHR₉ and R₉ is a C₁₋₅ alkoxycarbonyl or benzyloxycarbonyl group, which are referred to as the compounds of formula(Ih) hereinafter, can be prepared from the compounds of formula(Ie) by employing Process J given below: 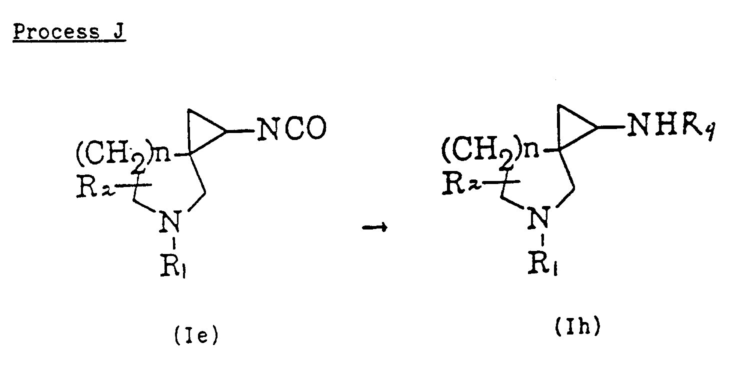
wherein:
- R₁, R₂ and n are: the same as defined in the formula(I); and
- R₉ is: a C₁₋₅ alkoxycarbonyl or benzyloxycarbonyl group.

In accordance with Process J, the compounds of the formula (Ih) can be prepared by reacting the compounds of formula(Ie) with an alcohol having 1 to 5 carbon atoms or benzyl alcohol in a non-reactive solvent such as toluene, benzene, xylene and the like, while heating. The above reaction can be carried out in the presence of a base such as triethylamine, sodium alkoxide and the like as a catalyst.

Further, the R₁ group in the formula(Ih) may be removed in the same manner as in Process H; and the compounds of formula(Ih) wherein R₁ is a hydrogen atom can be converted to the acid addition salts thereof with a conventional organic or inorganic acid.

The compounds of formula(I) wherein R₃ is -NR₉R₁₀, R₉ is a C₁₋₅ alkoxycarbonyl or benzyloxycarbonyl group and R₁₀ is a C₁₋₅ alkyl group, which are referred to as the compounds of formula (Ii), can be prepared from the compounds of formula(Ih) in accordance with the following Process K. 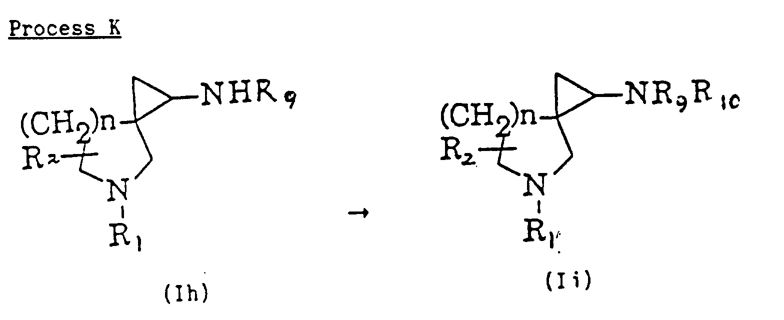
wherein:
- R₁, R₂ and n are: the same as defined in the formula(I);
- R₉ is: a C₁₋₅ alkoxycarbonyl or benzyloxycarbonyl group; and
- R₁₀ is: a C₁₋₅ alkyl group.

In accordance with Process K, said compounds of formula(Ih) can be reacted with the compounds represented by the formula of R₁₀-Hal wherein R₁₀ is the same as defined above and Hal is a halogen atom, in the presence of a base to give the compounds of formula(Ii). The base which may be employed in above reaction includes sodium hydride, potassium hydride and the like; and the suitable solvent for the reaction may include tetrahydrofuran, hexamethylphosphoramide, dimethylformamide, dimethylsulfoxide or a mixture thereof.

The R₁ group in the compounds of formula(Ii) thus obtained may be converted to a hydrogen atom, whereby the acid addition salts thereof may be obtained.

The compounds of formula(I) wherein R₃ is -NHR₁₀ and R₁₀ is a C₁₋₅ alkyl group, which are referred to as the compounds of formula(Ij) hereinafter, can be prepared from the compounds of formula(Ii) by using the following Process L. 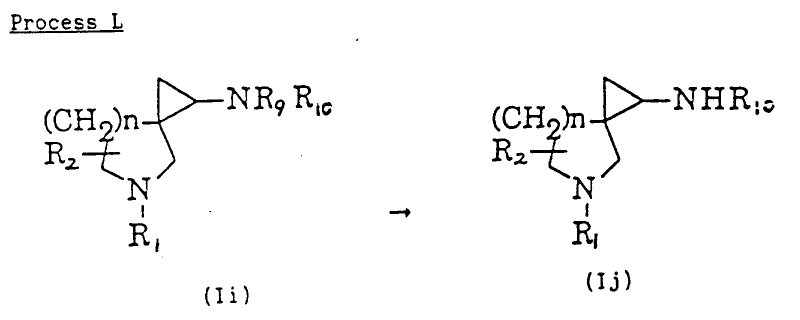
wherein:
- R₁, R₂ and n are: the same as defined in the formula(I),
- R₉ is: a C₁₋₅ alkoxycarbonyl or benzyloxycarbonyl group, and
- R₁₀ is: a C₁₋₅ alkyl group.

In Process L, said compounds of formula(Ij) can be prepared by hydrolyzing the compounds of formula(Ii) with an acid or alkali in the same manner as described in Process H.

The compounds of formula(I) wherein R₃ is a hydroxymethyl group, which are referred to as the compounds of formula(Ik) hereinafter, can be prepared from the compounds of formula(Ib) by employing the Process M given below: 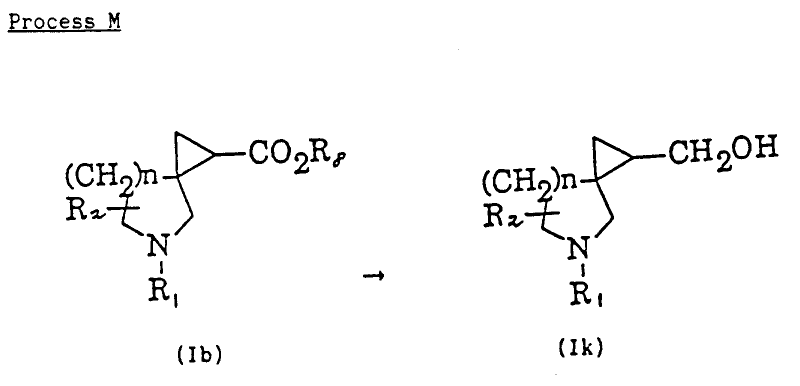
wherein:
- R₁, R₂ and n are: the same as defined in the formula(I), and
- R₈ is: a C₁₋₅ alkyl group.

In accordance with Process M, said compounds of formula(Ib) can be subjected to a reduction with a reducing agent such as lithium aluminum hydride, diisobutyl aluminum hydride, sodium borohydride-calcium chloride and the like to obtain the compounds of formula(Ik), with the proviso that the lithium aluminum hydride is used only for the compounds of formula(Ib) wherein R₁ is an optionally substituted benzyl group. The above reaction can be preferably carried out in an organic solvent such as tetrahydrofuran, ethyl ether, or a mixture thereof at a temperature of -10°C to the boiling point of the solvent used.

The R₁ group in the compounds of formula(Ik) thus obtained can be converted into a hydrogen atom in the same manner described in the Process H, whereby the acid addition salts thereof being obtained in accordance with the conventional method.

The compounds of formula(I) wherein R₃ is -(CH₂)mY, and m is 1 and Y is a C₂₋₅ acyloxy, mesyloxy, or p-toluensulfonyloxy group, which are referred to as the compounds of formula(Il) hereinafter, can be prepared from the compounds of formula(Ik) by using the following Process N. 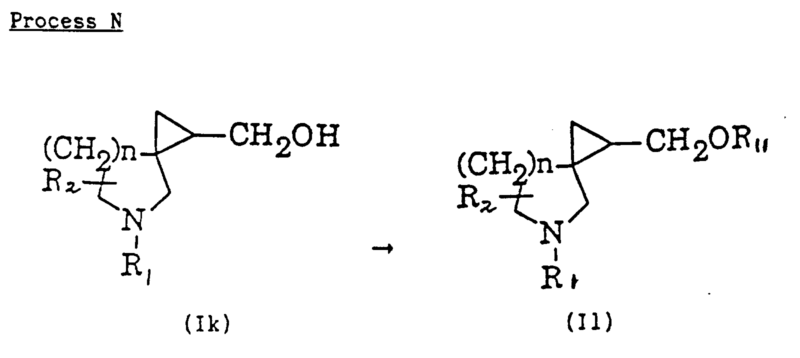
wherein:
- R₁, R₂ and n are: the same as defined in the formula(I), and
- R₁₁ is: a C₂₋₅ acyl, mesyl or p-toluenesulfonyl group.

In accordance with Process N, the compounds of formula(Ik) are reacted with the compounds of formula R₁₁-Hal wherein R₁₁ is the same as defined above and Hal is a halogen atom, or acid anhydride thereof, to produce the compounds of formula(Il). The above reaction can be carried out in the presence of a base such as triethylamine, diisopropylethylamine and the like in a solvent such as pyridine, dichloromethane or a mixture thereof.

The compounds of formula(I) wherein R₃ is an aminocarbonyl group which is optionally substituted with a C₁₋₈ alkyl or benzyl radical, which are referred to as the compounds of formula(Im), can be prepared from the compounds of formula(Ib) by using Process O given below: 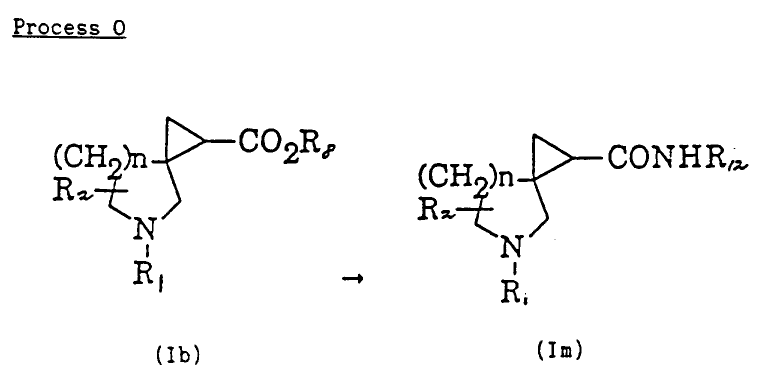
wherein:
- R₁, R₂ and n are: the same as defined in the formula(I), and
- R₁₂ is: a C₁-C₈ alkyl or benzyl group.

In accordance with Process O, the compounds of formula(Ib) are reacted with the amines of formula NH₂-R₁₂ wherein R₁₂ is the same as defined previously, at a normal pressure or in a sealed tube, while maintaining the temperature within the range from a room temperature to 250°C to produce the compounds of formula(Im).

The compounds of formula(I) wherein R₃ is -CH₂-NHR₁₂ and R₁₂ is a hydrogen atom, a benzyl or C₁₋₈ alkyl group, which are referred to as the compounds of formula(In) hereinafter, can be made from the compounds of formula(Im) as follows: 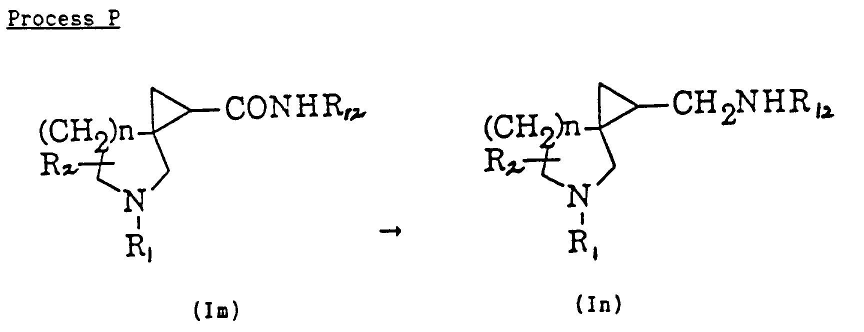
wherein:
- R₁, R₂ and n are: the same as defined in the formula (I), and
- R₁₂ is: a hydrogen atom, a C₁₋₈ alkyl or benzyl group.

In this process, the compounds of formula(In) are prepared by reduction of the compounds of formula(Im) with lithium aluminum hydride.

The above reaction can be normally conducted in a solvent such as tetrahydrofuran, ethyl ether or a mixture thereof, preferably at a temperature ranging from 0°C to the boiling point of solvent used.

The compounds of formula(In) thus obtained can be converted to those compounds wherein R₁ is a hydrogen atom by hydrogenation using palladium-on-carbon as a catalyst in methanol or ethanol at a pressure ranging from a normal pressure to 60 psi or by reduction in the presence of formic acid, acetic acid or hydrochloric acid.

The isomers can be resolved in accordance with the following scheme:
The following examples are intended to further illustrate the present invention, without limiting the scope of the invention.

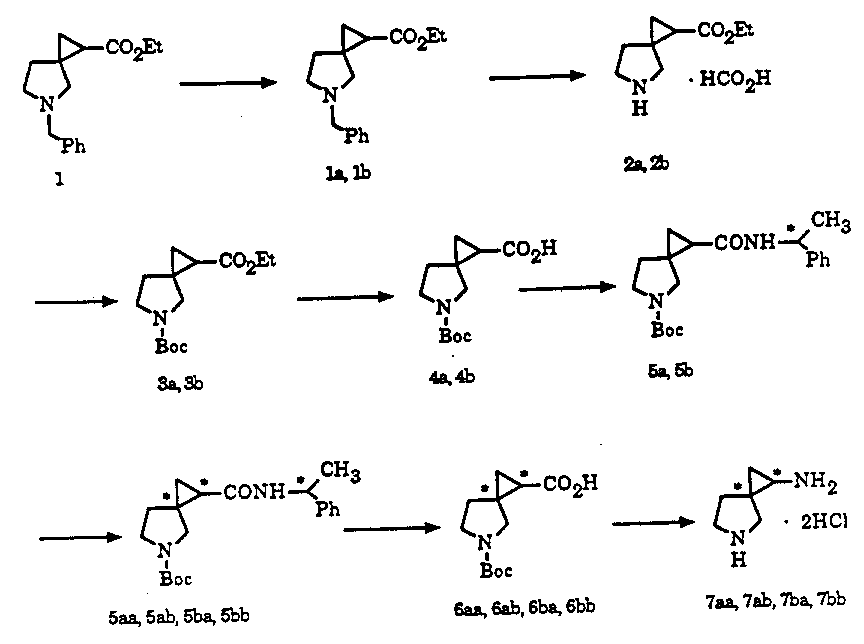

### Preparation Example 1: Synthesis of 1-benzyl-3-methylenepyrrolidine

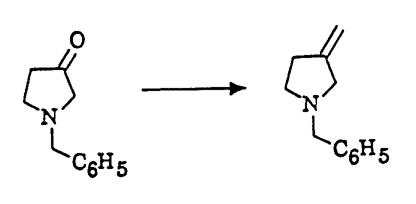

To a suspension of 150g(0.37mol) of methyltriphenylphosphonium iodide in 1ℓ of anhydrous tetrahydrofuran was added dropwise n-butyl lithium(2.5mol, 137mℓ of cyclohexane-mixed solution) at 0°C, while purging with a nitrogen gas; and the resulting mixture was stirred for 30 min at the same temperature and a solution of 30g(0.17mol) of 1-benzyl-3-pyrrolidone in 300mℓ of anhydrous tetrahydrofuran was added thereto. The reaction mixture was then stirred for one hour and a little of methanol was added. Thereafter, the solid thus formed was filtered and washed with 300mℓ of ethyl acetate. The filtrate and the washed solution were combined and concentrated. The residue was purified by employing of column chromatography on silica gel eluting with hexane: ethyl acetate=20:1 to afford 12.0g of the oily desired compounds(yield: 40.8%).
- ¹H-NMR (CDCl₃, δ ppm) :: 7.30 (5H, m), 4.85 (2H, m), 3.61 (2H, s), 2.68 (2H, m), 2.49 (2H, m)

### Preparation Example 2: Synthesis of 1-benzyloxycarbonyl-3-methylenepyrrolidine

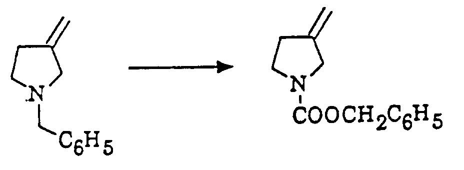

To a solution of 9g(52mmol) of 1-benzyl-3-methylenepyrrolidine in 20mℓ of anhydrous dichloromethane was added dropwise 15mℓ(0.1mol) of benzyl chlorocarbonate; and the resulting mixture was then stirred for 12 hours and 22mℓ(0.15mol) of benzylchloroformate was further added. After stirring for 6 hours, the reaction mixture was diluted with the addition of 20mℓ of dichloromethane. The diluted solution was washed with aqueous saturated sodium bicarbonate solution and saturated sodium chloride solution, dried over anhydrous magnesium magnesium sulfate and concentrated under a reduced pressure. The residue was subject to silica gel column chromatography eluting with 10:1 hexane: ethyl acetate to afford 5.5g of the oily desired compound (yield: 48.7%).
- ¹H-NMR (CDCl₃, δ ppm) :: 7.32 (5H, m), 5.20 (2H, s), 4.99 (2H, m), 4.02 (2H, m), 3.50 (2H, m), 2.50 (2H, m)

### Exampel 1: Synthesis of 5-benzyloxycarbonyl-3-ethyoxycarbonyl-5-azaspiro[2,4]heptane

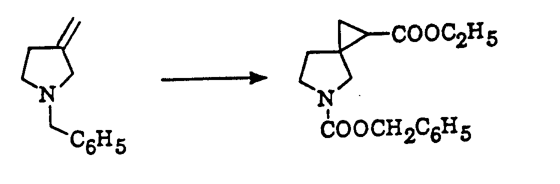

5.5g(25.3mmol) of 1-benzyloxycarbonyl-3-methylenepyrrolidine prepared in the preparation Exampel 2 and 55mg of rhodium acetate diner were added to 20mℓ of cyclohexane and the resultant was refluxed. To this solution was added dropwise a solution of 6.4mℓ (60.7mmol) of ethyldiazoacetate in 20mℓ of cyclohexane over 10 hours; the resultant was filtered and evaporated under a reduced pressure. The residue was purified by a silica gel column chromatography using 10:1 hexane:ethyl acetate to obtain 3.1g of the desired compound(yield: 40.4%).
- ¹H-NMR (CDCl₃, δ ppm) :: 7.42 (5H, m), 5.25 (2H, s), 4.22 (2H, q, J = 7.4 Hz), 3.61 (4H, m), 1.98 (3H, m), 1.37 (1H, m), 1.25 (3H, t, J = 7.4 Hz), 1.20 (1H, m)

### Exampel 2: Synthesis of 5-benzyloxycarbonyl-5-azaspiro[2,4]heptane-1-carboxylic acid

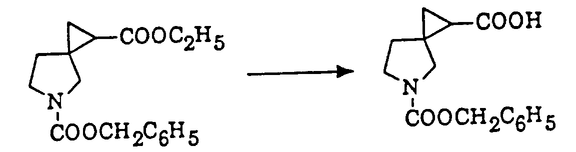

15g(49.9mmol) of 5-benzyloxycarbonyl-1-ethylcarbonyl-5-azaspiro[2,4]heptane was dissolved in 100mℓ of ethanol and 100mℓ of aqueous 5%-NaOH solution was added. The resultant was then stirred for 1.5 hours at a room temperature and concentrated under a reduced pressure. The concentrate thus obtained was diluted with the addition of 50mℓ of water and washed with 50mℓ of dichloromethane. The aqueous layer was acidified by adding an aqueous 2N hydrochloric acid solution with cooling, and extracted three times with 50mℓ portions of dichloromethane. The extracts were combined and dried over anhydrous magnesium sulfate and evaporated under a reduced pressure to give 12.4g of the desired compound(yield: 91.2%).
- ¹H-NMR (CDCl₃, δ ppm) :: 10.12 (1H, br, s), 7.35 (5H, m), 5.16 (2H, s), 3.05 (4H,), 1.98 (3H, m), 1.37(1H, t, J = 5 Hz), 1.22 (1H, dd, J = 8, 4.5Hz)

### Exampel 3: Synthesis of 5-benzyloxycarbonyl-1-azidocarbonyl-5-azaspiro[2,4]heptane

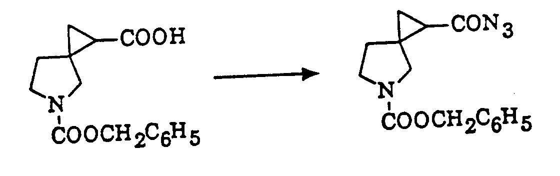

12g(43.6mmol) of 5-benzyloxycarbonyl-5-azaspiro[2,4]heptane-1-carboxylic acid was dissolved in 35mℓ of acetone and 4.9g (48.0mmol) of triethylamine was added dropwise for 5 minutes. Then, to the solution was added dropwise a solution of 5.2g(48.0mmol) of ethylchlorocarbonate in 15mℓ of acetone at -5°C to 0°C for 30 minutes and the resultant was further stirred for 15 minutes. A solution of 5.2g(87.7mmol) of sodium azide in 15mℓ of water was added dropwise at the same temperature over 20 minutes; and the resultant was stirred for an additional 30 minutes and cooled water was added. The resultant was extracted three times with 70mℓ portions of toluene; and the toluene layers formed were dried over magnesium sulfate and evaporated at lower temperature under a reduced pressure to afford 11.4g of the desired compound(yield: 87.2%).
- ¹H-NMR (CDCl₃, δ ppm) :: 7.33 (5H, m), 5.15 (2H, s), 3.07 (4H, m), 1.99 (3H, m), 1.37 (1H, t, J = 5 Hz), 1.23 (1H, dd, J = 8, 4.5 Hz)

### Example 4: Synthesis of 5-benzyloxycarbonyl-1-isocyanato-5-azaspiro [2,4]heptane

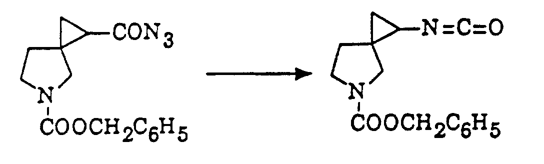

11.0g(36.7mmol) of 5-benzyloxycarbonyl-1-azidocarbonyl-5-azaspiro[2,4]heptane was dissolved in 200mℓ of anhydrous toluene; and the resulting solution was refluxed until the elution of nitrogen gas was ceased, and concentrated under a reduced pressure to obtain 9.5g of the desired oily compound(yield: 95.2%).
- ¹H-NMR (CDCl₃, δ ppm) :: 7.32 (5H, m), 5.24 (2H, s), 3.61 (4H, m), 1.98 (3H, m), 1.37 (1H, t, J = 5 Hz), 1.21 (1H, dd, J = 8, 4.5 Hz)

### Example 5: Synthesis of 1-amino-5-azaspiro[2,4]heptane hydrochloride

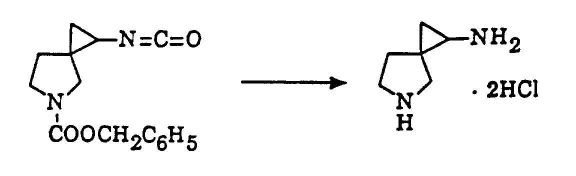

9.0g(33.1mmol) of 5-benzyloxycarbonyl-1-isocyanato-5-azaspiro[2,4]heptane was cooled and 60mℓ of 8N-hydrochloric acid solution was added thereto. Thereafter, the reaction solution was increasingly warmed, refluxed for 10 minutes and concentrated under a reduced pressure to 5.5g of the oily desired compound(yield: 89.8%).
- ¹H-NMR (CDCl₃, δ ppm) :: 3.30 (4H, m), 2.65 (1H, m), 1.91 (2H, m), 1.26 (1H, dd, J = 5.7 Hz), 1.12 (1H, t, J = 5 Hz)

### Preparation Example 3: Synthesis of 1-benzyl-3-methylene-pyrrolidine

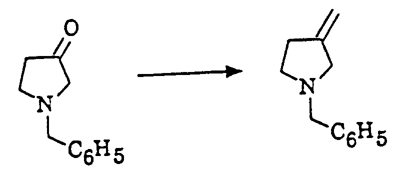

26.8g(100mmol) of methylene diiodide and 11.7g(180mmol) of zinc dust were added to 100mℓ of tetrahydrofuran and the solution was stirred under a nitrogen gas for 30 minutes. Then, to the solution was added 3.8g(20mmol) of titanium(IV) chloride at 0°C and the reaction mixture was stirred for 30 minutes at a room temperature. To this was then added dropwise 3.5g(20mmol) of 1-benzyl-3-pyrrolidone dissolved in 20mℓ of tetrahydrofuran; and the resultant was stirred for 1 hour at a room temperature. Thereafter, the reaction mixture was added to aqueous saturated sodium bicarbonate solution and the reaction product was extracted with ethyl acetate. The organic layer was dehydrated with anhydrous mirabilite and concentrated. The resulting residue was subjected to silica gel column chromatography using hexane: ethyl acetate 10:1(v/v) to obtain 0.24g of the desired compound in the form of liquid.
- ¹H-NMR (CDCl₃, δ ppm) :: 7.30 (5H, m), 4.85 (2H, m), 3.61 (2H, s), 2.68 (2H, m), 2.49 (2H, m)

### Preparation Example 4: Synthesis of 1-benzyl-3-(ethoxycarbonylmethylene)-1-pyrrolidine

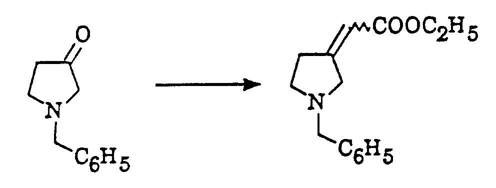

21g of sodium hydride(60 to 65%) was dispersed in 1ℓ of tetrahydrofuran; and the dispersion was cooled to -20°C under a nitrogen gas and 125g(0.588mol) of triethylphosphonoacetate was added thereto. The reaction mixture was then stirred at a room temperature, and when the elution of hydrogen was ceased, 85g(0.485mol) of 1-benzyl-3-pyrrolidone dissolved in 50mℓ of tetrahydrofuran was added. Thereafter, the reaction mixture was stirred at a room temperature for 2 hours, diluted with 1ℓ of ehtyl acetate and washed twice with water. The organic layer thus formed was dehydrated with anhydrous sodium sulfate and concentrated. The residue was passed over silica column eluting with a gradient of hexane/ethylacetate(20/1 → 5/1 → 2/1) to afford 96g of the desired compound in the form of liquid(yield: 80%).
- ¹H-NMR (CDCl₃, δ ppm) :: 7.25 (5H, m), 5.68 (1H, m), 4.07 (2H, m), 3.60 (3H, m), 3.17 (1H, br,s), 2.90 (1H, m), 2.64 (1H, t, J = 6.6 Hz), 2.57 (2H, s), 1.20 (3H, m)

### Example 6: Synthesis of 5-benzyl-1-ethoxycarbonyl-5-azaspiro[2,4] heptane

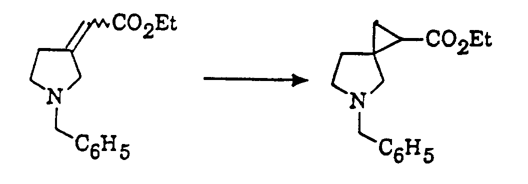

255g of trimethylsulfoxonium iodide was suspended in 1ℓ of dimethylsulfoxide and to the suspension was added 48g of sodium hydride(55 to 60%) under a nitrogen gas. The resultant was stirred at a room temperature until the elution of hydrogen gas did not observe, and then, a solution of 245g of 1-benzyl-3-(ethylcarbonylethylene)-1-pyrrolidine in 180mℓ of dimethylsulfoxide was added. The reaction mixture was stirred at a room temperature for 1 hour and at 55 to 60°C for 2 hours, and was cooled to a room temperature. To the cooled solution was added 2ℓ of water and the resultant was extracted four times with 700mℓ portions of dichloromethane. The organic layer was washed with brine, dehydrated with anhydrous sodium sulfate and concentrated. Then, the residue thus obtained was subjected to silica gel column chromatography to obtain 103g of the desired compound in the form of liquid(yield: 40%).
- MS m/z (rel. int.%) :: 259 (M⁺, 2.5), 230(21), 214(2), 159(40), 140(4), 91(29), 85(63), 83(100)
- ¹H-NMR (CDCl₃, δ ppm) :: 7.30 (5H, m), 4.10(2H, m), 3.63 (2H,s), 2.72∼1.7 (8H, m), 1.35∼1.05 (4H, m)

### Example 7: Synthesis of 5-benzyloxycarbonyl-1-ethyloxycarbonyl-5-azaspiro[2,4]heptane

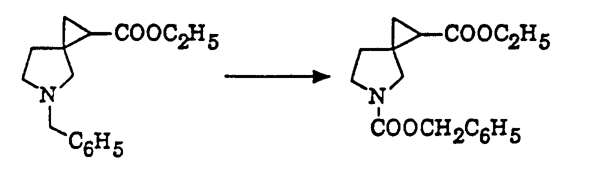

30g(0.12mole) of 5-benzyl-1-ethoxycarbonyl-5-azaspiro[2.4] heptane was dissolved in 60mℓ of anhydrous dichloromethane and 33mℓ of benzylchloroformate was added dropwise thereto at a room temperature. Thereafter, to the resultant was added 52mℓ of benzyl chlorocarbonate, and the mixture was further stirred for 6 hours and diluted with 50mℓ of dichloromethane. The resultant was then washed twice with 100mℓ portions of brine, dried over anhydrous magnesium sulfate and concentrated under a reduced pressure. The residue was subject to column chromatography eluting wiht hexane:ethyl acetate = 9:1 to obtain 15.5g of the oily desired compound(yield: 45%).
- ¹H-NMR (CDCl₃, δ ppm) :: 7.42 (5H, m), 5.25 (2H, s), 4.22 (2H, q, J = 7.4 Hz), 3.61 (4H, m), 1.98 (3H, m), 1.37 (1H, m), 1.25 (3H, t, J = 7.4Hz), 1.20 (1H, m)

### Example 8: Synthesis of bis-(1,5-ethoxycarbonyl)-5-azaspiro[2,4] heptane

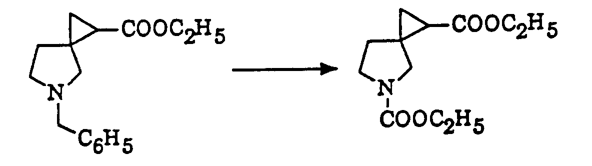

103g of 5-benzyl-1-ethoxycarbonyl-5-azaspiro[2,4]heptane was dissolved in 500mℓ of benzene and 180mℓ of ethyl chloroformate was added; and the resuting solution was heated under reflux for 8 hours. The reaction mixture was concentrated under a reduced pressure and the residue was subject to silica gel column chromatography to afford 65g of the desired compound as a yellow oil (yield: 68%).
- MS m/z (rel. int. %) :: 241(M⁺, 3.2), 196(9.7), 168(4), 154(3.8), 141(100), 113(15), 82(33), 68(47)
- ¹H-NMR (CDCl₃, δ ppm) :: 4.17∼4.10 (4H, m), 3.55∼3.3 (4H, m), 2.1∼1.7 (3H, m), 1.4∼1.1 (7H, m)

### Example 9: Synthesis of 5-ethoxycarbonyl-5-azaspiro[2,4]heptane-1-carboxylic acid

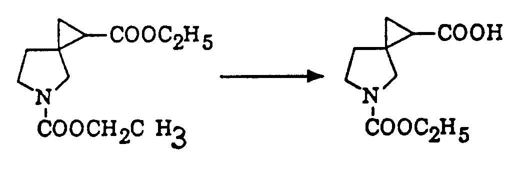

To a solution of 65g of bis-(1,5-ethoxycarbonyl)-5-azaspiro [2,4]heptane in 420mℓ of ethanol was added 420mℓ of aqueous 5% NaOH solution; and the resultant was stirred at a room temperature for 3 hours. The reaction mixture was then concentrated under a reduced pressure in water bath at 30°C and ethanol was distilled off. To the resulting solid was added 210mℓ of distilled water; the solution was acidifed to pH 3 to 4 by using 2N-HCl solution and extracted with 500mℓ of dichloromethane. The organic layer was washed with distilled water and brine, dried over anhydrous magnesium sulfate and concentrated under a reduced pressure to afford 49.5g of the desired compound as brown oil(yiled: 87%).
- MS m/z (rel. int. %) :: 213 (M⁺, 15), 168(10), 141(100), 113(11), 82(22), 68(25)
- ¹H-NMR (CDCl₃, δ ppm):: 4.13 (2H, q, J = 7 Hz), 3.6∼3.3 (5H, m), 2.15∼1.3 (4H, m), 1.25 (3H, t, J = 7 Hz)

### Example 10: Synthesis of 5-(t-butoxycarbonyl)-1-ethoxycarbonyl-5-azaspiro[2,4]heptane

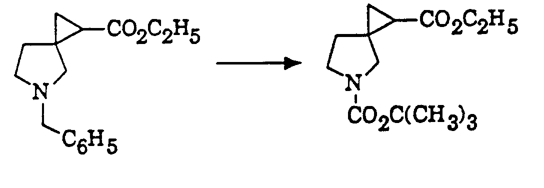

To a solution of 518mg of 5-benzyl-1-ethoxycarbonyl-5-azaspiro[2,4]heptane in 4.4% formic acid-methanol was added 300mg of 10% palladium on carbon; and the resultant was stirred under a nitrogen gas for 16 hours and filtered. To the filtrate thus obtained was 1.72mℓ of triethylamine and 650mg of di-t-butyldicarbonate and the mixture was then stirred at a room temperature for 16 hours. Thereafter, the reaction mixture was concentrated under a reduced pressure and the residue was subject to silica gel column chromatography to obtain 350mg of colorless oily material(yield: 68%).
- MS m/z (rel. int. %) :: 269(M⁺, 1), 212(5), 168(3), 113(10), 84(33), 69(25), 58(76)
- ¹H-NMR (CDCl₃, δ ppm) :: 4.1 (2H,m), 3.5∼3.35 (4H,m), 1.94 (1H,m), 1.72 (2H,m), 1.42 (9H,s), 1.35∼1.1 (5H,m)

### Example 11: Synthesis of 5-t-butoxycarbonyl-1-t-butoxycarbonylamino-5-azaspiro[2,4]heptane

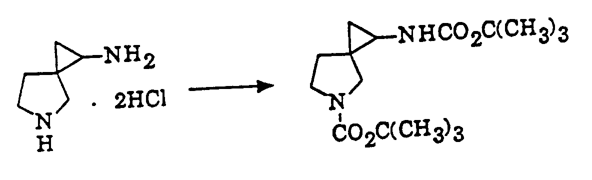

To a solution of 14g(75.5mmol) of 1-amino-5-azaspiro[2,4] heptane hydrochloride in 150mℓ of methanol was added 16.7g(165mmol) of triethylamine and 39.5g(181mmol) of di-t-butyldicarbonate at 0°C; and the resulting soultion was stirred at a room temperature for 1 hour and concentrated under a reduced pressure. The residue was dissolved in 100mℓ of ethylacetate, washed several with water, dried over magnesium sulfate and evaporated under a reduced pressure. The residue obtained was subject to column chromatography eluting with 10:1 hexane:ethyl acetate to provide 17.2g of the liquid desired compound(yield: 72.9%).
- ¹H-NMR (CDCl₃, δ ppm) :: 3.49 (2H, m), 3.22 (2H, m), 2.53 (1H, m), 1.82 (2H, m), 1.43 (18H, 2 x s), 1.12 (1H, t, J = 5 Hz), 0.97 (1H, dd, J = 5.7 Hz)

### Example 12: Synthesis of 1-t-butoxycarbonylmethylamino-5-t-butoxycarbonyl-5-azaspiro[2,4]heptane

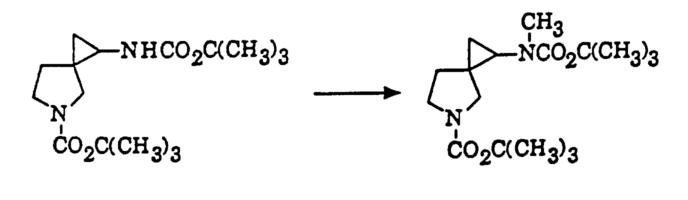

1g(3.2mmol) of 5-t-butoxycarbonyl-1-t-butoxycarbonyl amino-5-azaspiro[2,4]heptane was dissolved in 5mℓ of hexamethylphosphoramide and 0.17g(4.5mmol) of sodium hydride was added at 0°C. Then, the reaction mixture was stirred at a room temperature and 0.6g (4.2mmol) of methyl iodide was added at 0°C. After stirring for further 1hour, at a room temperture, to this solution was added 5mℓ of water. The resultant was extracted three times with 10mℓ of portions of dichloromethane, dried over magnesium sulfate anhydride and concentrated under a reduced pressure. The residue thus obtained was subjected to silica gel column chromatography eluting with 10:1 hexane:ethyl acetate to afford 0.67g of the desired compound(yield: 64.1%).
- ¹H-NMR (CDCl₃, δ ppm) :: 3.49 (2H, m), 3.22 (2H, m), 2.75 (3H, s), 2.59 (1H, m), 1.43 (18H, 2 x s), 1.12 (1H, t, J = 5 Hz), 0.96 (1H, dd, J = 5.7 Hz)

### Example 13: Synthesis of 1-methylamino-5-azaspiro[2,4]heptane hydrochloride

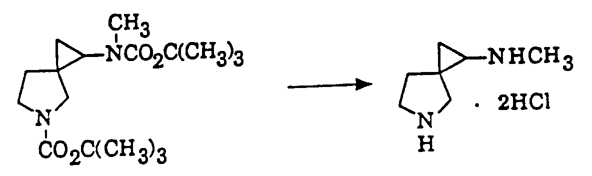

To 50mℓ of 2N-hydrochloric acid-methanol solution was added 3.26g(10mmol) of 1-t-buotxycarbonylmethylamino-5-t-butoxycarbonyl-5-azaspiro[2,4]heptane and the resulting solution was stirred at a room temperature for 5 hours and concentrated under a reducedc pressure to obtain 1.63g of the desired compound(yield: 82%).
MS m/z : 126(M⁺)
- ¹H-NMR (D₂O, δ ppm) :: 3.32 (4H, m), 2.63 (1H, m), 2.42 (3H, s), 1.90 (2H, m), 1.25 (1H, dd, J = 5,7Hz), 1.12 (1H, t, J = 5Hz)

### Preparation Example 5: Synthesis of 1-benzyl-3-(ethoxycarbonylmethylene)-1-piperidine

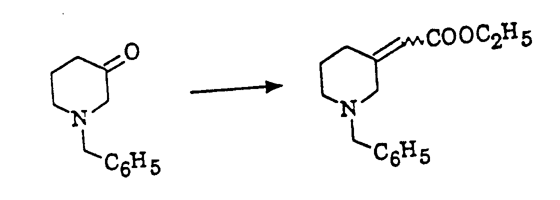

5.3g of sodium hydride(60 to 65%) was dispersed in 200mℓ of tetrahydrofuran; and the dispersion was cooled to -20°C and 24.2g(108mmol) of triethyl phosphonoacetate was added dropwise. The resultant was stirred at a room temperature, and when the elution of hydrogen gas was ceased, a solution of 17g(90mmol) of 1-benzyl-3-piperidone in 20mℓ of tetrahydrofuran was added thereto. Thereafter, the reaction mixture was stirred at a room temperature for 2 hours, diluted with 200mℓ of ethyl acetate and washed twice with 400mℓ portions of water. The organic layers thus separated were dried over magnesium sulfate and concentrated. The residue was subjected to silica gel column chromatography eluting with hexane:ethyl acetate =8:1 to afford 19.1g of the desired compound in the form of liquid (yield:82%).
- ¹H-NMR (CDCl₃, δ ppm) :: 7.28 (5H, m), 5.62 (1H, 2 x s), 4.11 (2H, 2 x q, J = 7.1 Hz), 3.68 (1H, s), 3.60 (1H, s), 3.52 (1H, s), 2.91 (1H, s), 2.82 (1H, m), 2.50 (2H, m), 2.20 (1H, m), 1.70 (2H, m), 1.21 (3H, 2 x g, J = 7.1 Hz)

### Example 14: Synthesis of 5-benzyl-1-(ethoxycarbonyl)-5-azaspiro[2,5] octane

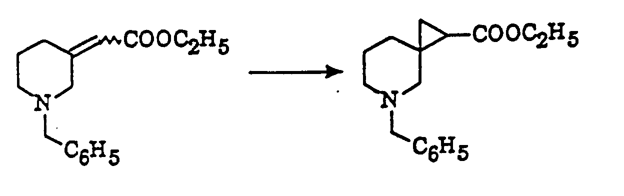

25.5g(0.116mol) of trimethylsulfoxonium iodide was suspended in 150mℓ of dimethyl sulfoxide and 5.5g of sodium hydride (55∼60%) was added under a nitrogen gas. The resulting solution was then stirred at a room temperature until the generation of hydrogen gas did not observe, and a solution of 25g(0.096mol) of 1-benzyl-3-(ethoxycarbonylmethylene)-1-piperidine in 30mℓ of dimethylsulfoxide was added. Thereafter, the reaction mixture was stirred for 1 hour at a room temperature and for 1 hour at 55 to 60°C, and cooled to a room temperature; and 200mℓ of water was added. The resultant was extracted four times with 200mℓ portions of dichloromethane; and the organic layers were washed with brine, dried over magnesium sulfate and concentrated. The residue thus obtained was subjected to silica gel column chromatography eluting with hexane:ethyl acetate=5:1 to afford 5.8g of the liquid desired compound(yield: 21.3%).
- ¹H-NMR (CDCl₃, δ ppm) :: 7.27 (5H, m), 4.11 (2H, q, J = 4.6 Hz), 3.46 (2H, s), 2.49∼2.12 (4H, m), 1.69 (1H, m), 1.55∼1.45 (4H, m), 1.21 (3H, t, J = 4.6 Hz), 1.08 (1H, t, J = 4.6 Hz), 0.84 (1H, dd, J = 4.6, 7.9 Hz)

### Example 15: Synthesis of 5-benzyloxycarbonyl-1-ethyloxycarbonyl-5-azaspiro[2,5]octane

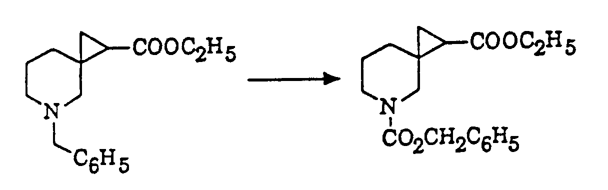

5.6g(20.5mmol) of 5-benzyl-1-ethoxycarbonyl-5-azaspiro [2,5]octane was dissolved in 20mℓ of anhydrous dichloromethane and 50.6mℓ of benzylchloroformate was added dropwise thereto at a room temperature; and the resulting solution was stirred for 12 hours and additional 8.8mℓ of benzylchlroformate was added dropwise. Then, the reaction mixture was further stirred for 6 hours, diluted with 10mℓ of dichloromethane, washed twice with 50mℓ portions of aqueous saturated sodium bicarbonate solution and with a brine, dried over anhydrous magnesium sulfate and concentrated under a reduced pressure. The residue was subjected to silica gel column chromatography eluting with hexane:ethyl acetate=9:1 to obtain 3.8g of the oily desired compound(yield:67.9%).
- ¹H-NMR (CDCl₃, δ ppm) :: 7.31 (5H, m), 5.08 (2H, s), 4.08 (2H, q, J = 5.32 Hz), 3.59∼3.22 (4H, m), 1.74 (1H, m), 1.65 (2H, m), 1.97 (2H, m), 1.22 (3H, t, J = 5.32 Hz), 1.56 (1H, t = 4.6 Hz), 0.83 (1H, dd, J = 4.6, J = 7.9 Hz)

### Example 16: Synthesis of 5-benzyloxycarbonyl-5-azasprio[2,5]octane-1-carboxylic acid

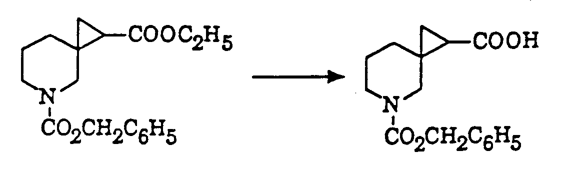

6g(13.2mmol) of 5-benzyloxycarbonyl-1-ethoxycarbonyl-5-azaspiro[2,5]octane was dissolved in 20mℓ of ethanol and 20mℓ of aqueous 5% sodium hydroxide solution was added, which was stirred for 3 hours at a room temperature and concentrated under a reduced pressure. The resultant was diluted with 10mℓ of water and washed with 20mℓ of dichloromethane; and the aqueous layer thus separated was acidified with 2N-hydrochloric acid under cooling and triple-extracted with 20mℓ portions of dichloromethane. Then, the extracts were combined, dried over anhydrous magnesium sulfate and concentrated under a reduced pressure. The residue was subjected to silica gel column chromatography eluting with chloroform:methanol=20:1 to afford 3g of the desired compound[yield: 92.8%).
- MS m/z :: 289 (M⁺)
- ¹H-NMR (CDCl₃, δ ppm) :: 7.82 (5H, m), 5.11 (2H, s), 3.70∼3.27 (4H, m), 1.80 (1H, dd, J = 4.9, 7.9 Hz), 1.68∼1.40 (4H, m), 1.11 (1H, t, J = 4.0 Hz), 0.88 (1H, dd, J = 4.6, 7.9 Hz)

### Example 17: Synthesis of 1-amino-5-azaspiro[2,5]octane hydrochloride

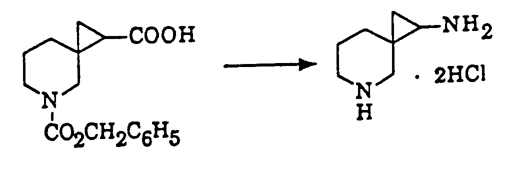

2.9g(11.8mmol) of 5-benzyloxycarbonyl-5-azaspiro[2,5] octane-1-carboxylic acid was dissolved in 10mℓ of acetone and 1.4g (13.7mmol) of triethylamine was added dropwise over 5 min; and to the resulting mixture was added dropwise a solution of 1.5g(13.8mmol) of ethylchloroformate in 5mℓ of acetone at -5°C to 0°C for 30 min, which was stirred for 15 min. To this solution was added dropwise a solution of 1.4g(23.7mmol) of sodium azide in 5mℓ of water at the same temperature for 20 min; and the resultant was further stirred for 30 min and cooled water was added. Then, the resultant was extracted three times with 15mℓ portions of toluene, and the toluene layers were dried over anhydrous magnesium sulfate and refluxed until the elution of nitrogen gas did not observe. The resultant was then concentrated under a reduced pressure and 15mℓ of 8N-hydrochloric acid was added with cooling, increasingly heated, refluxed for 10 min, and concentrated under a reduced pressure to give 1.5g of the oily desired compound(yield: 63.8%).
- MS m/z (rel. int., %) :: 126 (M⁺, 9.6), 109 (6.7), 83 (100)
- ¹H-NMR (CDCl₃, δ ppm) :: 3.42 (4H, m), 2.61 (1H, m), 1.68∼1.40 (4H, m), 1.21 (1H, t, J = 4.0 Hz), 1.11 (1H, dd. J = 4.6, 7.9 Hz)

### Example 18: Synthesis of 5-benzyl-1-benzylamido-5-azaspiro[2,4] heptane

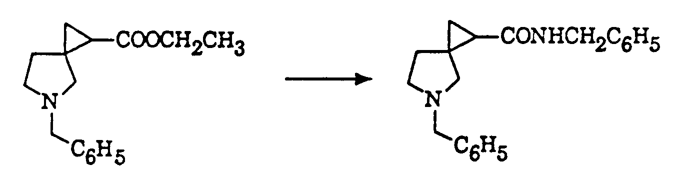

13g of 5-benzyl-1-ethoxycarbonyl-5-azaspiro[2,4]heptane was mixed with 10g of benzylamine; and the mixture was heated under a reflux for 4 days and benzylamine only was distilled off under a reduced pressure. The residue was subjected to silica gel column chromatography eluting with 2 to 10% methanol-chloroform to obtain 9.36g of the desired compound(yield: 58%).
- Mass m/z (rel. int., %) :: 377 (M⁺, 22), 360 (95), 345 (82), 333 (25), 316 (45), 301 (100)
- ¹H-NMR (CDCl₃, δ ppm) :: 7.31 (10H, m), 6.25, 6.15 (1H, two, t), 4.5 (2H, m), 3.60 (2H, m), 2.8∼2.4 (4H, m), 2.02∼1.80 (2H, m), 1.50∼1.30 (2H, m), 1.01∼0.92 (1H,m)

### Example 19: Synthesis of 5-benzyl-1-benzylaminomethyl-5-azaspiro[2,4] heptane

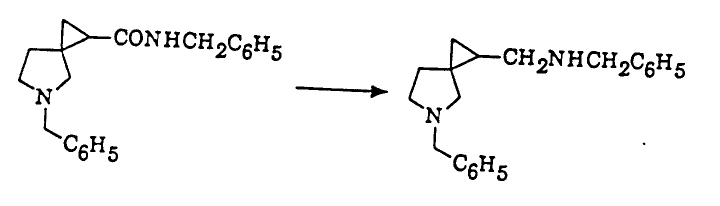

2g of lithium aluminum hydride was added to 50mℓ of tetrahydrofuran under a nitrogen gas; and to the resulting solution was a solution of 9.2g of 5-benzyl-1-benzylamido-5-azaspiro[2,4] heptane in 70mℓ of tetrahydrofuran was added dropwise in the ice water bath, which was then stirred for 3 hours at a room temperature and heated under a reflux for 3 hours. The reaction mixture was cooled with ice water and 10mℓ of water and 10mℓ of 30% sodium hydroxide solution were added; and the resultant was diluted with 20mℓ of methanol and 150mℓ of dichloromethane and filtered through a celite. The filtrate was washed with water and the organic layer was dried with anhydrous sodium sulfate and concentrated. The residue thus obtained was subjected to silica gel column chromatography eluting with 2 to 10% methanol-chloroform to afford 6.7g of the desired compound(yield: 76%).
- ¹H-NMR (CDCl₃, δ ppm) :: 730 (10H, m), 3.75 (2H, sextet), 3.61 (2H, q), 2.70∼2.35 (8H, m), 1.81 (1H, t), 1.01 (1H, m), 1.70 (1H, dd, J = 4.7 Hz), 0.27 (1H, m)

### Example 20: Synthesis of 1-aminomethyl-5-azaspiro[2,4]heptane formate

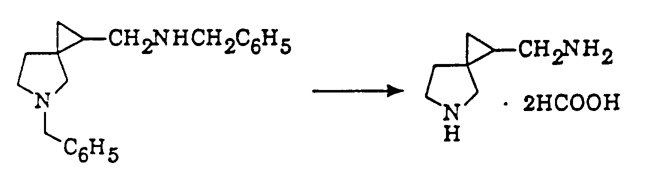

4.1g of 5-benzyl-1-benzylaminomethyl-5-azaspiro[2,4] heptane was dissolved in 120mℓ of ethanol and 1.5mℓ of 98% formic acid and 2g of 10% palladuim on carbon were added; and the resultant was agitated for 16 hours at 50 psi hydrogen pressure and then filtered through a celite. The filtrate was concentrated to produce 2.4g of the syrupy desired compound (yield: 82%).
- Mass m/z (rel. int., %) :: 126 (M⁺, 25), 109 (100), 94 (25), 82 (45), 68 (95)
- ¹H-NMR (DMSO-d₆, δ ppm) :: 3.87∼3.55 (4H, m), 3.21 (1H, m), 2.90 (1H, m), 2.00∼1.87 (2H, m), 1.87 (1H, m), 1.05 (1H, dd, J = 8.5, 5.4 Hz), 0.73 (1H, t, J = 5.4 Hz)

### Example 21: Synthesis of 1-hydroxymethyl-5-benzyl-5-azaspiro[2,4] heptane

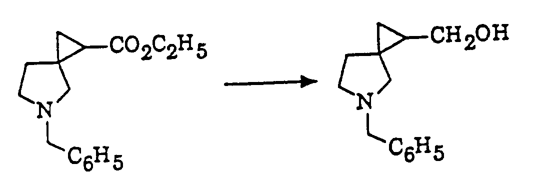

200mg of lithium aluminum hydride was dispersed in 10mℓ of tetrahydrofuran under a nitrogen gas and a solution of 770mg of 5-benzyl-1-ethoxycarbonyl-5-azaspiro[2,4]heptane in 10mℓ of tetrahydrofuran was added dropwise, which was then heated under reflux for 2 hours. The reaction mixture was then cooled to a room temperature and 1mℓ of water and 2mℓ of aqueous 20% sodium hydroxide solution were added, which was stirred for 30 min and filtered. The filtrate was washed with a little of water, dried with anhydrous sodium sulfate and concentrated to 434mg of the desired compound (yield: 68%).
- ¹H-NMR (CDCl₃, δ ppm) :: 7.31 (5H, m), 3.80∼3.55 (4H, m), 3.35 (1H, m), 2.83 (1H, m), 2.72 (1H, m), 2.45 (1H, m), 2.1∼1.65 (2H, m), 1.12 (1H, m), 0.76 (1H, m), 0.45 (1H, m)

### Example 22: Synthesis of 1-hydroxymethyl-5-azaspiro[2,4]heptane formate

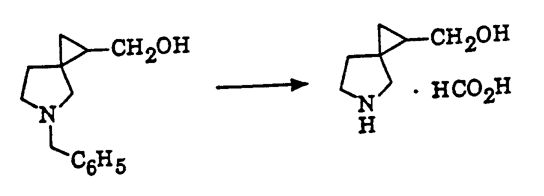

400mg of 1-hydroxymethyl-5-benzyl-5-azaspiro[2,4]heptane was dissolved in 10mℓ of ethanol and 1mℓ of 98% formic acid and 100mg of 10% palladium on carbon were added. The resultant was then allowed to stand for 15 hours at 50psi and filtered; and the filtrate was concentrated to 290mg of the desired compound(yield: 91%).
- ¹H-NMR (DMSO-d₆, δ ppm) :: 3.85∼3.60 (2H, m), 3.40 (1H, m), 2.80 (1H, m), 2.71 (1H, m), 2.46 (1H, m), 2.00∼1.62 (2H, m), 1.13 (1H, m), 0.75 (1H, m), 0.47 (1H, m)

### Example 23: Synthesis of 1-hydroxy-5-azaspiro[2,4]heptane hydrochloride

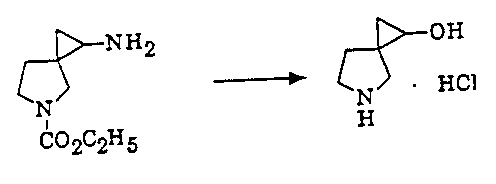

920mg of 1-amino-5-ethyoxycarbonyl-5-azaspiro[2,4]heptane was dissolved in 20mℓ of 3N-hydrochloric acid and a solution of 500mg of sodium nitrite in 5mℓ of water was added dropwise at 0°C; and the reaction mixture was stirred for 2 hours at the same temperature. To the resultant was then added 5mℓ of conc. hydrochloric acid and the mixture was stirred at a room temperature for 1 day. The solvent was evaporated under a reduced pressure and the portions which were soluble in chloroform-methanol(3:1) solution was taken and centrated. The concentrate was dissolved in a minimum amount of water, and 20mℓ of ethanol and ethylether were added; the precipitate thus formed was filtered and dried under a reduced pressure to 388mg of the desired compound(yield: 52%).
- ¹H-NMR (D₂O, δ ppm) :: 3.35 (4H, m), 2.69 (1H, m), 1.90 (2H, m), 1.20 (1H, dd, J = 5.7 Hz), 1.10 (1H, t, J = 5Hz)

### Example 24: Synthesis of 1-acetoxymethyl-5-t-butoxycarbonyl-5-azaspiro[2,4]heptane

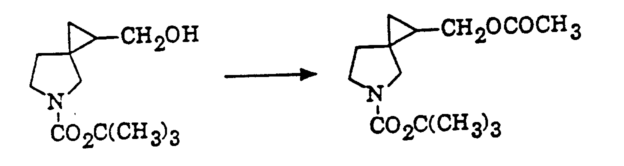

454mg of 1-hydroxymethyl-5-t-butoxycarbonyl-5-azaspiro [2,4]heptane was dissolved in 5mℓ of pyridine and 2mℓ of acetic acid anhydride was added; and the solution was stirred at a room temperature for 20 hours. An excess of reagent and solvent was evaporated under a reduced pressure; and the residue was dissolved in 20mℓ of dichloromethane and washed with water. The organic layer was then dried over anhydrous mirabilite and concentrated to 495mg of the desired compound(yield: 92%).
- ¹H-NMR (DMSO-d₆, δ ppm) :: 3.91∼3.60 (2H, m), 3.34 (1H, m), 2.80 (1H, m), 2.70 (1H, m), 2.45 (1H, m), 2.20 (3H, s), 2.00∼1.64 (2H, m), 1.45 (9H, s), 1.10 (1H, m), 0.77 (1H, m), 0.50 (1H, m)

### Example 25: Resolution of 5-benzyl-1-ethoxycarbonyl-5-azaspiro[2,4] heptane isomers(1a, 1b)

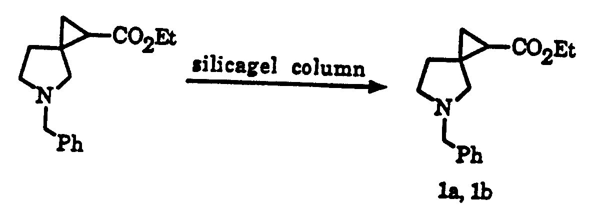

224mg of 5-benzyl-1-ethoxycarbonyl-5-azaspiro[2,4]heptane was subjected to silica gel column chromatography eluting with 5∼30% hexane:ethyl acetate(1:1) to obtain 48g of the compound of formula(Ia) having Rf value of 0.5 and 95g of the compound of formula(Ib) having Rf value of 0.45.
- ¹H-NMR (CDCl₃, δ ppm) for 1a :: 1.09 (1H, dd, J = 8.4, 4.5 Hz), 1.23 (3H, t, J = 4.9 Hz), 1.23 (1H, t, J = 4.5 Hz), 1.66 (1H, dd, J = 8.4, 5.5 Hz), 2.40 (1H, d, J = 9.3), 2.57 (1H, d, J = 9.3 Hz), 2.71 (2H, m), 3.58 (2H, m), 4.10 (2H, m), 7.30 (5H, m)
- ¹H-NMR (CDCl₃, δ ppm) for 1b :: 1.09 (1H, dd, J = 8.3, 4.5 Hz), 1.24 (3H, t, J = 4.9 Hz), 1.28 (1H, t, J = 4.5 Hz), 1.71 (1H, dd, J = 8.3, 5.5 Hz), 1.88 (2H, m), 2.38 (4H, m), 3.61 (2H,s), 4.11 (2H, m), 7.30 (5H, m)

### Example 26: Synthesis of 5-(t-butoxycarbonyl)-1-ethoxycarbonyl-5-azaspiro[2,4]heptane(3a)

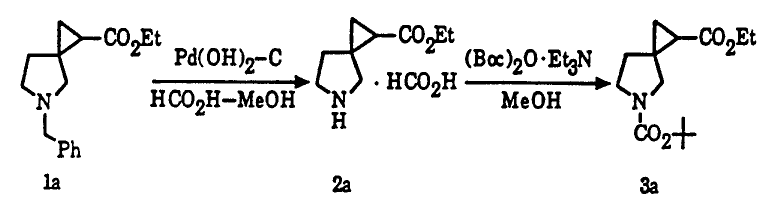

46g of 5-benzyl-1-ethoxycarbonyl-5-azaspiro[2,4]heptane(1a) was dissolved in 300mℓ of methanol; and 14mℓ of 98% formic acid and 12g of 20% Pd(OH)₂-Carbon were added. The resultant was stirred at a room temperature for 40 hours under hydrogen stream and filtered through celite. The filtrate was concentrated to obtain a compound of formula(2a) in an oily material. The obtained compound was dissolved in 200mℓ of methanol; and 28mℓ of triethylamine was added. Then, 43g of di-t-butyldicarbonate was added dropwise and the resultant was stirred for 16 hours at a room temperature, warmed in a water bath at 40°C for 1 hour and concentrated to remove the solvent. The residue was subjected to silica gel column chromatography eluting with 5∼30% acetate-hexane to obtain 30g of the desired compound of formula(3a).
- ¹H-NMR (CDCl₃, δ ppm) :: 1.23 (3H, t, J = 7.2 Hz), 1.23 (1H, dd, J = 8.1, 5.3 Hz), 1.41 (1H, t, J = 4.8), 1.97 (1H, m), 2.21 (2H, m), 3.37 (4H, m), 4.18 (2H, m)

### Example 27: Synthesis of 5-(t-butoxycarbonyl)-1-ethoxycarbonyl-5-azaspiro[2,4]heptane(3b)

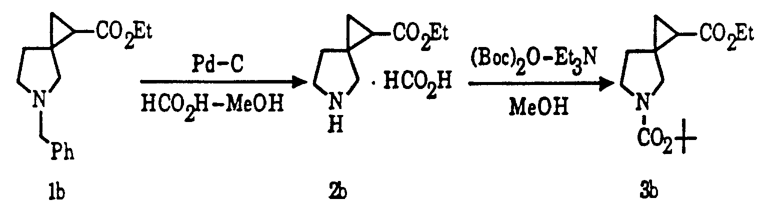

92.6g of 5-benzyl-1-ethoxycarbonyl-5-azaspiro[2,4]heptane (1b) was dissolved in 950mℓ of 4.4% formic acid-methanol; and 11g of 20% Pd(OH)₂-Carbon and 20g of 10% Pd-C were added. The catalyst was removed under hydrogen stream; and the filtrate was concentrated to obtain a compound of formula(2b). The obtained compound was dissolved in 500mℓ of methanol; and 57mℓ of triethylamine was added. Then, 86g of di-t-butyldicarbonate was added dropwise and the resultant was stirred for 15 hours at a room temperature, warmed in a water bath at 50°C for 2 hours and concentrated to remove the solvent. The residue was subjected to silica gel column chromatography eluting with 5∼30% ethylacetate-hexane to obtain 69.6g of the desired compound of formula (3b, yield 72.3%).
- ¹H-NMR (CDCl₃, δ ppm) :: 1.18 (1H, m) 1.19 (3H, t, J = 7.1 Hz), 1.31 (1H, t, J = 5.3 Hz) 1.83 (1H, dd, J = 8.1, 5.6 Hz), 1.93 (2H, m), 3.40 (4H, m), 4.08 (2H, m)

### Example 28: Synthesis of 5-(t-butoxycarbonyl)-1-ethoxycarbonyl-5-azaspiro [2,4] heptane (3a, 3b)

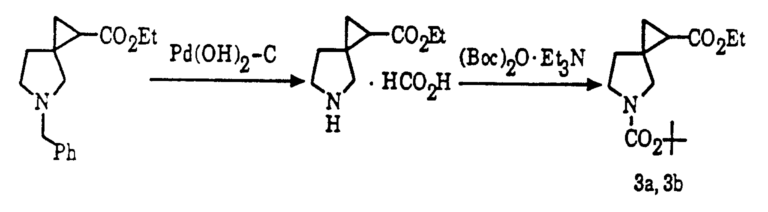

50g of 5-benzyl-1-ethoxycarbonyl-5-azaspiro [2,4] heptane was dissolved in 400mℓ of 4.4% formic acid-methanol; and 10g of 20% Pd(OH)₂-Carbon was added. The resultant was stirred at a room temperature for 24 hours under hydrogen stream and filtered through celite to remove the catalyst; and the filtrate was concentrated. The obtained residue was dissolved in 300mℓ of methanol; and 30mℓ of triethylamine was added. Then, 45g of di-t-butyldicarbonate was added dropwise and the resultant was stirred for 24 hours at a room temperature and concentrated to remove the solvent. The residue was subjected to silica gel column chromatography eluting with 5∼30% ethylacetate-hexane to obtain 6g of the desired compound of formula (3a) and 15g of the desired compound of formula(3b).

### Example 29: Synthesis of 5-(t-butoxycarbonyl)-5-azaspiro [2,4] heptane carboxylic acid(3a)

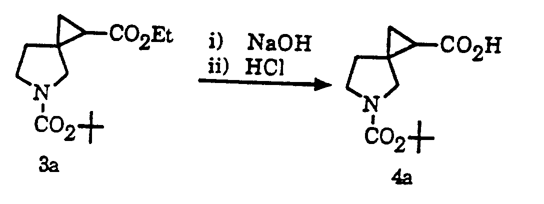

29g of 5-(t-butoxycarbonyl)-1-ethoxycarbonyl-5-azaspiro [2,4] heptane (3a) was dissolved in 150mℓ of methanol; and a solution of 10g of sodium hydroxide dissolved in 150mℓ of water was added. The resultant was stirred at a room temperature for 3 hours and concentrated at a temperature of not more than 40°C to remove methanol. The resulting solid was cooled with ice water, acidified to pH 4 by using 2N-HCl solution and extracted three times with 300mℓ of dichloromethane. The extract was combined, dehydrated with anhydrous sodium sulfate and concentrated to afford 26.6g of the desired compound of formula(4a).
- ¹H-NMR (CDCl₃, δ ppm) :: 1.17 (1H, dd. J = 8.3, 4.7 Hz), 1.31 (1H, t, J = 4.7 Hz), 1.39 (9H, s), 1.76 (1H, m), 2.00 (2H, m), 3.25 (2H, m), 3.43 (2H, m)

### Example 30: Synthesis of 5-(t-butoxycarbonyl)-5-azaspiro[2,4]heptane-1-carboxylic acid(4b)

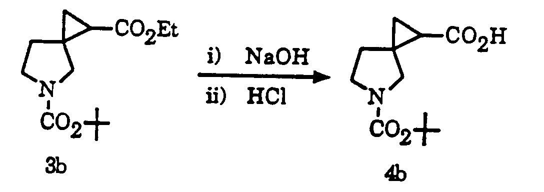

69.2g of 5-(t-butoxycarbonyl)-1-ethoxycarbonyl-5-azaspiro [2,4]heptane(3b) was dissolved in 450mℓ of methanol; and a solution of 20g of sodium hydroxide dissolved in 200mℓ of water was added. The resultant was stirred at a room temperature for 5 hours and concentrated at a temperature of not more than 40°C to remove methanol. To the resulting solution was added 100mℓ of water; the solution was acidified to pH 4 by using 3N-HCl solution, left over night at a room temperature and filtered to recover the produced precipitate. The filtrate was extracted with dichloromethane, concentrated and dried under a reduced pressure to obtain 58.5g of the desired compound(yield 94%).
- ¹H-NMR (CDCl₃, δ ppm) :: 1.17 (1H, dd, J = 8.3. 4.7 Hz), 1.38 (1H, t, J = 4.7 Hz), 1.45 (9H, s), 1.72 (2H, m), 1.97 (1H, m), 3.45 (2H, m)

### Example 31: Synthesis of N-[1-(R)-phenylethyl]-5-(t-butoxycarbonyl)-5-azaspiro [2,4] heptane-1-carboxamide (5aa, 5ab)

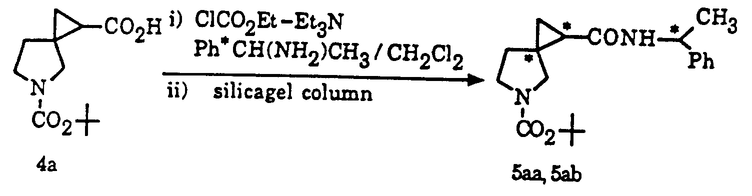

26.3g of 5-(t-butoxycarbonyl)-5-azaspiro[2,4]heptane-1-carboxylic acid (4a) was dissolved in 200mℓ of dichloromethane; and 20mℓ of triethylamine was added. To the resulting solution was added dropwise 13mℓ of ethylchloroformate diluted in 50mℓ of dichloromethane at a temperature of -5°C; and the resultant was stirred at said temperature for 1 hour. A solution of 18.8g of R-(+)-α-methyl benzylamine dissolved in 50mℓ of dichloromethane was added dropwise at a temperature of -5°C and stirred at said temperature for 2 hour and at a room temperature for 3 hour. The reaction mixture was washed with 300mℓ of water three times; and the organic layer was dehydrated over magnesium sulfate. The residue was subjected to silica gel column chromatography eluting with 10% isopropylalcohol-10~60% ethylacetate:hexane (2:1) to obtain 16.3g of the desired compound of formula (5aa) (Rf 0.5) and 14.8g of formula (5ab).
- ¹H-NMR (CDCl₃, δ ppm) for 5aa :: 0.96 (1H, dd, J = 8.3, 4.7 Hz), 1.31 (1H, broad t, J = 4.7 Hz), 1.42 (9H, s), 1.44 (3H, d, J = 6.9 Hz), 1.49 (1H, m); 2.03 (2H, m), 3.19 (2H, m), 3.40 (2H, m), 5.10 (1H, q, J = 6.9 Hz), 6.02 (1H, m), 7.27 (5H, m)
- ¹H-NMR (CDCl₃, δ ppm) for 5ab :: 1.00 (1H, dd, J = 8.3, 4.7 Hz), 1.35 (1H, t, J = 4.7 Hz), 1.45 (9H, s), 1.47 (3H, d, J = 6.9 Hz), 1.51 (1H, m), 1.90 (2H, m), 3.19 (2H, m), 3.32 (2H, m), 5.11 (1H, d, J = 6.9 Hz), 6.03 (1H, m), 7.27 (5H, m)

### Example 32: Synthesis of N-[1-(R)-phenylethyl]-5-(t-butoxycarbonyl)-5-azaspiro[2,4]heptane-1-carboxamide(5ba, 5bb)

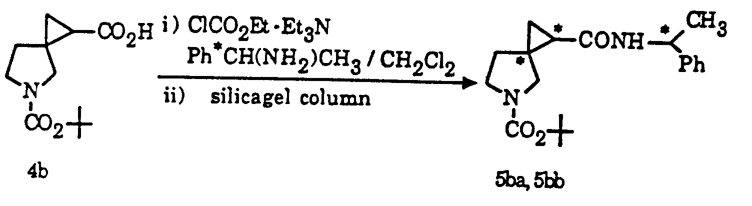

58.4g of 5-(t-butoxycarbonyl)-5-azaspiro[2,4]heptane-1-carboxylic acid(4b) was dissolved in 350mℓ of dichloromethane; and 40mℓ of triethylamine was added. To the resulting solution was added dropwise 27mℓ of ethylchloroformate diluted in 50mℓ of dichloromethane and the resultant was stirred at a room temperature over night, The reaction mixture was washed with 400mℓ of water three times; and the organic layer was dehydrated over magnesium sulfate. The residue was subjected to silica gel column chromatography eluting with 1% isopropylalcohol-10∼60% ethylacetate:hexane (2:1) to obtain 37g of the desired compound of formula(5ba)(Rf 0.45) and 33g of formula (5bb).
- ¹H-NMR (CDCl₃, δ ppm) for 5ba :: 1.11 (1H, dd, J = 8.1, 4.5 Hz), 1.37 (1H, t, J = 4.5 Hz), 1.37 (1H, t, J = 4.5 Hz), 1.44 (9H, s), 1.51 (3H, d, J = 6.87 Hz), 1.60 (1H, m), 1.89 (1H, m), 3.43 (4H, m), 5.6 (1H, q, J = 6.87 Hz), 5.94 (1H, m), 7.32 (5H, m)
- ¹H-NMR (CDCl₃, δ ppm) for 5bb :: 1.10 (1H, dd, J = 8.1, 4.5 Hz), 1.32 (1H, t, J = 4.5 Hz), 1.41 (9H, s), 1.48 (3H, d, J = 6.87 Hz), 1.62 (1H, m), 1.75 (1H, m), 3.40 (4H, m), 5.10 (1H, q, J = 6.87 Hz), 5.92 (1H, broads), 7.79 (5H, m)

### Example 33: Synthesis of (1S, 3S)-5-(t-butoxycarbonyl)-5-azaspiro [2,4] heptane-1-carboxylic acid (6aa)

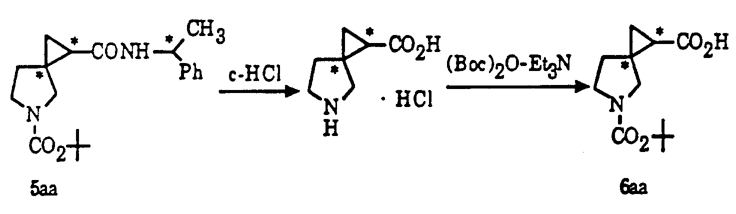

13.3g of (1S, 3S)-N-[1-(R)-phenylethyl]-5-(t-butoxycarbonyl)-5-azaspiro [2,4] heptane-1-carboxamide (5aa) was dissolved in 250mℓ of concentrated hydrochloric acid; and the resultant was heated under reflux for 5 hours and washed with 200mℓ of chloroform three times. The aqueous layer was concentrated under a reduced pressure. The residue was dissolved in 230mℓ of ethanol and 115mℓ of triethylamine; and 17g of di-t-butyldicarbonate was added thereto. The resultant was stirred at a room temperature for 16 hours and concentrated under a reduced pressure, 100mℓ of water was added; and the resulting solution was acidified to pH 4 by using N-HCl and extracted with 150mℓ of chloroform three times. The combined extract was concentrated; and the residue was subjected to silica gel column chromatography eluting with 2∼10% methanol-chloroform to obtain 5.8g of the desired compound of formula(6aa, yield 63%). m.p: 148 to 149°C
- Ms. m/z (rel. int. %) :: 241 (M⁺, 57), 186 (11), 168 (20), 113 (79), 69 (51), 57 (100)
- ¹H-NMR (CDCl₃, δ ppm) :: 1.17 (1H, dd, J = 8.3, 4.7 Hz), 1.30 (1H, broad t, s = 4.7 Hz), 1.75 (1H, m), 2.01 (2H, m), 3.25 (2H, m), 3.42 (2H, m)

### Example 34: Synthesis of (1R,3R)-5-(t-butoxycarbonyl)-5-azaspiro[2,4] heptane-1-carboxylic acid(6ab)

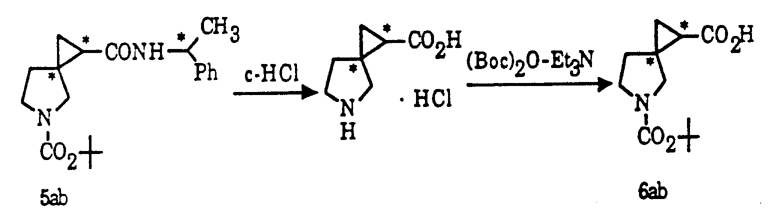

10.8g of (1R,3R)-N-[1-(R)-phenylethyl]-5-(t-butoxycarbonyl)-5-azaspiro[2,4]heptane-1-carboxamide(5ab) was dissolved in 215mℓ of concentrated hydrochloric acid; and the resultant was heated under reflux for 5 hours and washed with 200mℓ of chloroform three times. The aqueous layer was concentrated under a reduced pressure. The residue was dissolved in 185mℓ of ethanol and 93mℓ of triethylamine; and 13.7g of di-t-butyldicarbonate was added thereto. The resultant was stirred at a room temperature for 16 hours and concentrated under a reduced pressure. 100mℓ of water was added; and the resulting solution was acidified to pH 4 by using N-HCl and extracted with 200mℓ of chloroform three times. The combined extract was concentrated; and the residue was subjected to silica gel column chromatography eluting with 1∼10% methanolchloroform to obtain 5.8g of the desired compound of formula(6ab, yield 76%).
m.p: 143 to 145°C
- Ms. m/z (rel int. %) :: 241 (M⁺, 5), 186 (11), 168 (20), 113 (79), 69 (51), 57 (100)
- ¹H-NMR (CDCl₃, δ ppm) :: 1.17 (1H, dd, J = 8.3, 4.7 Hz), 1.30 (1H, broad t, J = 4.7 Hz), 1.39 (9H, s), 1.75 (1H, m), 2.01 (2H, m), 3.25 (2H, m), 3.42 (2H, m)

### Example 35: Synthesis of (1S,3R)-5-(t-butoxycarbonyl)-5-azaspiro [2,4]heptane-1-carboxylic acid(6ba)

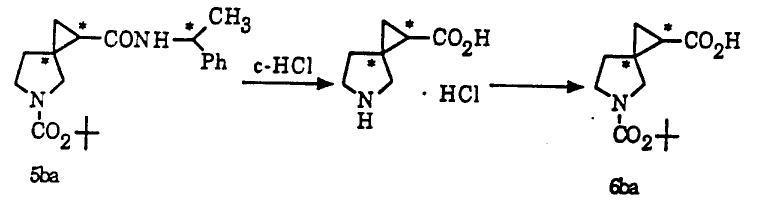

19g of (1S,3R)-N-[1-(R)-phenylethyl]-5-(t-butoxycarbonyl)-5-azaspiro[2,4]heptane-1-carboxamide(5ba) was dissolved in 250mℓ of concentrated hydrochloric acid; and the resultant was refluxed for 5 hours; washed with 200mℓ of chloroform four times and concentrated under a reduced pressure. The residue was dissolved in 150mℓ of methanol; and 16mℓ of triethylamine was added thereto. To the resultant was added portionwise 25g of di-t-butyldicarbonate at a room temperature. The resultant was stirred for 16 hours and concentrated under a reduced pressure. The residue was dissolved in a small amount of methanol; and 100mℓ of 4% NaOH solution was added. The resulting solution was stirred and washed with 50mℓ of dichloromethane four times. The aqueous layer was acidified to pH 4 by using 3N-HCl solution, extracted with 100mℓ of dichloromethane four times and concentrated. The residue was subjected to silica gel column chromatography to obtain 7.9g of the desired compound of formula(6ba)(yield 62.6%).
mp: 132 to 133°C
- Ms. m/z (rel. int. %) :: 241 (M⁺, 4), 186 (9), 168 (18), 113 (80), 57 (100), 41 (25)
- ¹H-NMR (CDCl₃, δ ppm) :: 1.18 (1H, dd, J = 8.3, 4.7 Hz), 1.39 (1H, t, J = 4.7 Hz), 1.45 (9H, s), 1.71 (2H, m), 1.98 (1H, m), 3.45 (2H, m)

### Example 36: Synthesis of (1R,3S)-5-(t-butoxycarbonyl)-5-azaspiro [2,4]heptane-1-carboxylic acid(6bb)

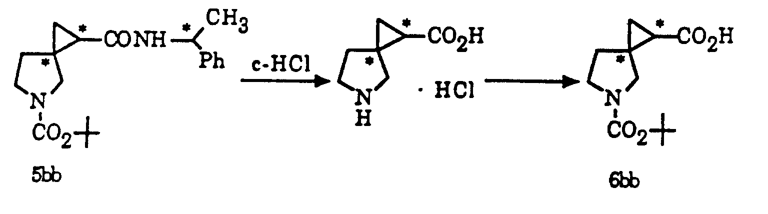

18g of (1R,3S)-N-[1-(R)-phenylethyl]-5-(t-butoxycarbonyl)-5-azaspiro[2,4]heptane-1-carboxamide(5bb) was dissolved in 200mℓ of concentrated hydrochloric acid; and the resultant was refluxed for 5 hours, washed with 200mℓ of chloroform four times and concentrated under a reduced pressure. The residue was dissolved in 200mℓ of methanol; and 16mℓ of triethylamine was added thereto. To the resultant was added portionwise 23g of di-t-butyldicarbonate at a room temperature. The resultant was stirred for 16 hours and concentrated under a reduced pressure. The residue was dissolved in a small amount of methanol; and 100mℓ of 4% NaOH solution was added. The resulting solution was stirred and washed with 50mℓ of dichloromethane four times. The aqueous layer was acidified to pH 5 by using 3N-HCl solution, extracted with 100mℓ of dichloromethane four times and concentrated. The residue was subjected to silica gel column chromatography to obtain 8.4g of the desired compound of formula (6bb) (yield 66.6%).
mp: 130 to 134°C
- Ms. m/z (rel. int. %) :: 241 (M⁺, 4), 186 (10), 168 (19), 113 (78), 57 (100), 41 (24)
- ¹H-NMR (CDCl₃, δ ppm) :: 1.15 (1H, dd, J = 8.3, 4.7 Hz), 1.32 (1H, t, J = 4.7 Hz), 1.42 (9H, s), 1.72 (2H, m), 1.95 (1H, m), 3.45 (4H, m)

### Example 37: Synthesis of (1S,3S)-1-amino-5-azaspiro-[2,4]heptane hydrochloride(7aa)

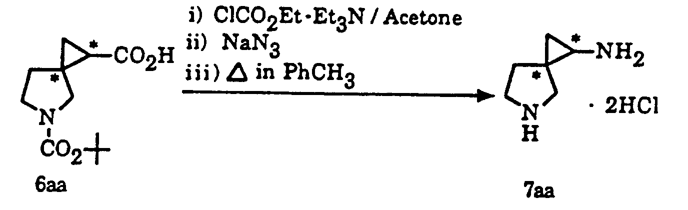

5.5g of(1S,3S)-5-(t-butoxycarbonyl)-5-azaspiro[2,4] heptane-1-carboxylic acid (6aa) was dissolved in 50mℓ of acetone; and 4.5mℓ of triethylamino was added thereto. To the resultant was added dropwise a solution of 3mℓ of ethylchloroformate diluted in 10mℓ of acetone at a temperature of -5°C over 15 minutes. The resulting solution was stirred for 30 minutes; and a solution of 3.7g of sodium azide dissolved in 10mℓ of water was added dropwise at said temperature over 20 minutes. After stirring for 30 minutes, 50mℓ of water was added. The resultant was extracted with 50mℓ of toluene three times; and the combined extract was dehydrated over anhydrous magnesium sulfate, heated under reflux until the elution of nitrogen gas was ceased and concentrated under a reduced pressure. To the residue was added 50mℓ of 6N-HCl solution; and the reaction mixture was stirred for 5 hours at a room temperature, refluxed for 10 mimutes and washed with 50mℓ of chloroform three times. The aqueous layer was concentrated under a reduced pressure to obtain 3.3g of the desired compound of formula(7aa)(yield 78.2%).
- Ms. m/z (rel. int. %) :: 112 (M⁺, 20), 83 (48), 70 (100), 43 (40), 36 (88)
- ¹H-NMR (D₂O, δ ppm) :: 1.02 (1H, dd, J = 7.7, 4.7 Hz), 1.21 (1H, t, J = 7.7 Hz), 1.98-2.10 (2H, m), 2.79 (1H, dd, J = 8.3, 4.7 Hz), 3.10 (1H, d, J = 12.4 Hz), 3.25 (1H, d, J = 12.4 Hz), 3.42 (2H, m)

### Example 38: Synthesis of(1R,3R)-1-amino-5-azaspiro-[2,4]heptane hydrochloride(7ab)

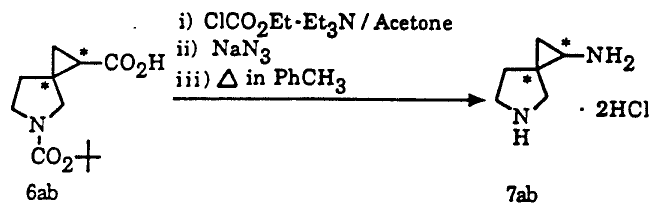

3.7g of(1R,3R)-5-(t-butoxycarbonyl)-5-azaspiro[2,4]heptane-1-carboxylic acid (6ab) was dissolved in 45mℓ of acetone; and 42.6mℓ of triethylamine was added thereto. To the resultant was added dropwise a solution of 1.8mℓ of ethylcholroformate diluted in 10mℓ of acetone at a temperature of -5°C over 15 minutes. The resulting solution was stirred for 30 minutes; and a solution of 2g of sodium azide dissolved in 10mℓ of water was added dropwise at said temperature over 20 minutes. After stirring for 30 minutes, 40mℓ of water was added. The resultant was extracted with 50mℓ of toluene three times; and the combined extract was dehydrated over anhydrous magnesium sulfat, heated under reflux until the elution of nitrogen gas was ceased and concentrated under a reduced pressure. To the residue was added 50mℓ of 6N-HCl solution; and the reaction mixture was stirred for 4 hours at a room temperature, refluxed for 10 mimutes and washed with 60mℓ of chloroform three times. The aqueous layer was concentrated under a reduced pressure to obtain 1.96g of the desired compound of formula (7ab) (yield 69.0%).
- Ms. m/z (rel. int. %) :: 112 (M⁺, 20), 83 (48), 70 (100), 43 (40)
- ¹H-NMR (CDCl₃, δ ppm) :: 1.02 (1H, dd, J = 7.7, 4.7 Hz), 1.21 (1H, t, J = 7.7 Hz), 1.98-2.10 (2H, m), 2.79 (1H, dd, J = 8.3, 4.7 Hz), 3.10 (1H, d, J = 12.4 Hz), 3.15 (1H, d, J = 12.4 Hz), 3.42 (2H, m)

### Example 39: Synthesis of (1S,3R)-1-amino-5-azaspiro-[2,4]heptane hydrochloride(7ba)

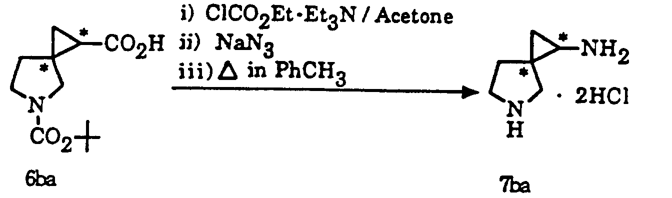

14g of(1S,3R)-5-(t-butoxycarbonyl)-5-azaspiro[2,4]heptane-1-carboxylic acid (6ba) was dissolved in 150mℓ of acetone; and 11.3mℓ of triethylamine was added thereto. To the resultant was added dropwise a solution of 7.73mℓ of ethylcholroformate diluted in 25mℓ of acetone at a temperature of -5°C over 60 minutes. To the reaction mixture was added dropwise a solution of 9.4g of sodium azide dissolved in 30mℓ of water over 40 minutes. After stirring for 1 hour 200mℓ of water was added. The resultant was extracted with 100mℓ of toluene three times; and the combined extract was dehydrated over anhydrous magnesium sulfate, heated under reflux until the elution of nitrogen gas was ceased and concentrated under a reduced pressure. To the residue was added 125mℓ of 8N-HCl solution; and the reaction mixture was stirred for 16 hours at a room temperature, heated under reflux for 20 minutes and washed with 100mℓ of chloroform three times. The aqueous layer was dehydrated with charcoal and concentrated under a reduced pressure to obtain 9g of the desired compound of formula (7ba) (yield 86%).
- Ms. m/z (rel. int. %):: 112 (M⁺, 20), 83 (48), 70 (100), 43 (40), 36 (88)
- ¹H-NMR (D₂O, δ ppm) :: 1.02 (1H, dd, J = 7.7, 4.7 Hz), 1.21 (1H, t, J = 7.7 Hz), 1.82∼2.02 (2H, m), 2.68 (1H, dd, J = 8.1, 4.7 Hz), 3.30 (2H, d, J = 2.8 Hz), 3.75∼3.41 (2H, m)

### Example 40: Synthesis of(1R,3S)-1-amino-5-azaspiro-[2,4]heptane hydrochloride(7bb)

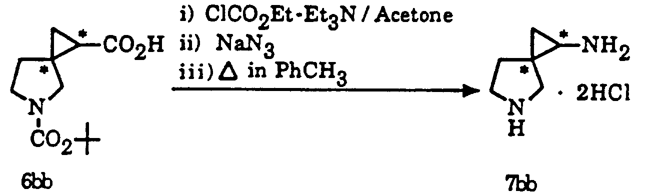

8.3g of(1R, 3S)-5-(t-butoxycarbonyl)-5-azaspiro[2,4] heptane-1-carboxylic acid (6bb) was dissolved in 150mℓ of acetone; and 6.3mℓ of triethylamino was added thereto. To the resultant was added dropwise mℓ of ethylcholroformate at a temperature of -5°C. The resulting solution was stirred for 1 hour at said temperature; and a solution of 5.6g of sodium azide dissolved in 30mℓ of water was added dropwise. After stirring for 1.5 hours, 300mℓ of water was added. The resultant was extracted with 100mℓ of toluene three times; and the combined organic layer was washed with 200mℓ of water three times, dehydrated over anhydrous magnesium sulfate, heated under reflux until the elution of nitrogen gas was ceased and concentrated under a reduced pressure. To the residue was added 120mℓ of 6N-HCl solution; and the reaction mixture was stirred for 3 hours at a room temperature, refluxed for 30 minutes and washed with 100mℓ of chloroform four times. The aqueous layer was concentrated under a reduced to obtain 5.4g of the desired compound of formula (7bb) (yield 85%)
- Ms. m/z (rel. int. %) :: 112 (M⁺, 20), 83 (48), 70 (100), 43 (37), 36 (88)
- ¹H-NMR (D₂O, δ ppm) :: 1.02 (1H, dd, J = 7.7, 4.7 Hz), 1.21 (1H, t, J = 7.7 Hz), 1.82∼2.02 (2H, m), 2.68 (1H, dd, J = 8.1, 4.7 Hz), 3.30 (2H, d, J = 2.8 Hz), 3.75∼3.41 (2H, m)

### Use Example 1: Synthesis of 7-(1-amino-5-azaspiro[2,4]heptane-5-yl)-1-cyclopropyl-6,8-difluoro-1,4-dihydro-4-oxoquinoline-3-carboxylic acid(Compound 1)

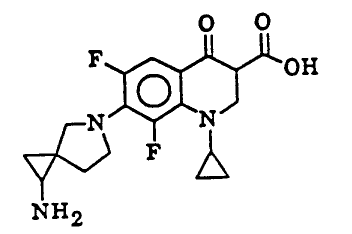

1.28g(4.52mmol) of 1-cyclopropvl-6,7,8-trifluoro-1,4-dihydro-4-oxoquinoline-3-carboxylic acid, 1g(5.40mmol) of 1-amino-5-azaspiro[2,4]heptane hydrochloride and 2.4g(15.76mmol) of 1,8 diazabicyclo[5,4,0]undec-7-ene were dissolved in 10mℓ of acetonitrile; and the resulting solution was refluxed for 3 hours and stirred for 1 hour. The precipitate produced was filtered, washed with chilled acetonitrile, water and ethylether in turn and dried under a reduced pressure to obtain 1g of the desired compound(yield: 58.9%).
- ¹H-NMR (DMSO-d₆, δ ppm) :: 8.59 (1H, s), 7.68 (1H, dd, J = 14.1, 1.6 Hz), 4.07 (1H, m), 3.82 (3H, m), 3.67 (1H, m), 2.28 (1H, dd, J = 7.2, 4.8 Hz), 1.86 (1H, m), 1.67 (1H, m), 1.15 (4H, m), 0.78 (1H, dd, J = 7.2, 4.5 Hz), 0.37 (1H, t, J = 4.5 Hz)
mp: 217°C

### Use Example 2: Synthesis of 7-(1-amino-5-azaspiro[2,5]octane-5-yl)-1-cyclopropyl-6,8-difluoro-1,4-dihydro-4-oxoquinoline-3-carboxylic acid(Compound 2)

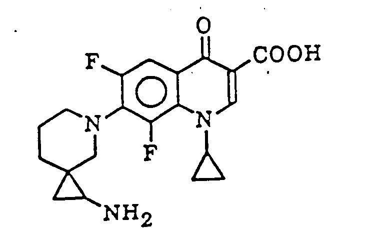

0.23g(1.2mmol) of 1-amino-5-azaspiro[2,5]octane hydrochloride, 0.28g(1mmol) of 1-cyclopropyl-6,7,8-trifluoro-1,4-dihydro-4-oxoquinoline-3-carboxylic acid and 0.46g(3mmol) of 1,8-diazabicyclo[5,4,0]undec-7-ene were dissolved in 3mℓ of acetonitrile; and the resulting solution was refluxed for 3 hours and cooled to room temperature. The precipitate produced was filtered, washed with acetonitrile, water and ethylether in turn and dried under a reduced pressure to obtain 0.18g of the desired compound (yield: 40%).
mp: 275∼277°C
- ¹H-NMR (CDCl₃, δ ppm) :: 8.70 (1H, s), 7.71 (1H, d, J = 11.8 Hz), 3.98 (1H, m), 3.11 (1H, d, J = 12.5 Hz), 2.86 (1H, d, J = 12.5 Hz), 2.34 (1H, dd, J = 4.0, 7.3 Hz), 1.86∼1.65 (4H, m), 1.32∼1.12 (4H, m), 0.62 (1H, dd, J = 5.2, 7.3 Hz), 0.25 (1H, t, J = 4.5 Hz)

### Use Example 3: Synthesis of 7-(1-aminomethyl-5-azaspiro[2,4]heptane-5-yl)-1-cyclopropyl-6,8-difluoro-1,4-dihydro-4-oxoquinoline-3-carboxylic acid(Compound 3)

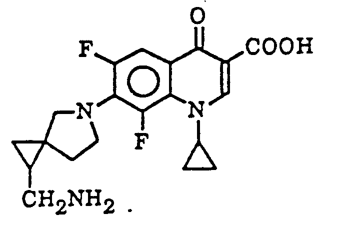

610mg of 1-aminomethyl-5-azaspiro [2,4] heptane diformate, 580mg of 1-cyclopropyl-6,7,8-trifluoro-1,4-dihydro-4-oxoquinoline-3-carboxylic acid and 750mg of 1,8-diazabicyclo [5,4,0] undec-7-ene were dissolved in 11mℓ of acetonitrile; and the resulting solution was heated under reflux and left at a room temperature over night. The precipitate produced was filtered, washed with acetonitrile, water, ethalol and ethylether in turn and concentrated under a reduced pressure to obtain 570mg of the desired compound (yield: 69%).
- ¹H-NMR (CDCl₃+CD₃COOD, δ ppm) :: 8.75, 8.69 (1H, two s), 7.75 (1H, d, J = 13.5 Hz), 4.05 (1H, m), 3.95∼3.65 (4H, m), 3.20 (1H, m), 2.92 (1H, m), 2.05∼1.85 (2H, m), 1.85 (1H, m), 1.30∼1.15 (4H, m), 1.00 (1H, dd, J = 8.5, 5.4 Hz), 0.70 (1H, t, J = 5.4 Hz)
The quinolone carboxylic acids compound prepared in the Use Examples by using several compounds according to the present invention were tested at below:

In order to demonstrate the superior effectiveness of the quinoline derivatives, the minimal inhibitory concentration(MIC) of several compounds synthesized in Use Examples hereof against the standard strains was determined and compared with ofloxacin. These MIC values were taken by employing two-fold dilution method: that is, two-fold serial dilutions of each of the test compounds were made and dispersed in a Mueller-Hinton agar medium; a standard strain which had the value of 10⁷ CFU/mℓ was inoculated on the medium, which was then incubated at 37°C for 18 hours. The results of the MIC tests are shown in the following Table.

**Table**

| In vitro anti-bacterial activity test against the standard strains(MIC: µg/mℓ) | | | | |
|---|---|---|---|---|
| Strain | Compound 1 | Compound 2 | Compound 3 | Ofloxcain |
| Streptococcus pyogenes 308A | 0.195 | 0.781 | 0.781 | 3.125 |
| Streptococcus faecium 77A | 0.098 | 0.391 | 0.391 | 1.563 |
| Staphylococcus aureus 285 | 0.025 | 0.098 | 0.195 | 0.391 |
| Staphylococcus aureus 503 | 0.025 | 0.098 | 0.195 | 0.391 |
| Escherichia coli DC 0 | 0.004 | 0.049 | 0.049 | 0.013 |
| Pseudomonas aeruginosa 1771M | 0.195 | 0.781 | 0.391 | 0.391 |
| Salmonella typhimurium | 0.013 | 0.049 | 0.098 | 0.025 |
| Klebsiella aerogenes 1522E | 0.013 | 0.195 | 0.098 | 0.049 |
| Enterobacter cloacae P 99 | 0.013 | 0.049 | 0.195 | 0.025 |

As can be seen from the result, the quinolone carboxylic acid derivatives possess a broad spectrum of potent anti-bacterial activities against Gram-positive and -negative bacteria, particularly Staphylococcus aureus as compared with the known ofloxacin.

## Claims

1. An azaspiro derivative of formula(I) and pharmaceutically acceptable salts thereof: 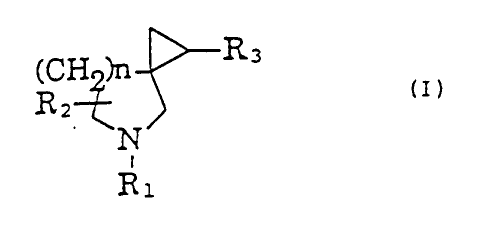 wherein:
R₁ is a hydrogen atom, an optionally substituted C₁₋₂₀ alkyl, optionally substituted benzyl, or nitrogen protecting group;
R₂ is a hydrogen atom or a methyl group which is preferably substituted at the 4- or 6-position carbon;
R₃ is -CO₂H, -CON₃, -NCO, -CONHR₁₀ wherein R₁₀ is a hydrogen atom, a benzyl, C₁₋₈ alkyl, or C₁₋₅ alkoxycarbonyl group, or -(CH₂)mY wherein m is 0 or 1 and Y is a hydroxyl, C₂₋₅ acyloxy, mesyloxy, p-toluensulfonyloxy or amino group which is optionally substituted with one or two C₁₋₄ alkyl radicals, with one C₁₋₄ alkanoyl radical, with one benzyl radical or with one nitrogen protecting radical metabolizable in vivo; and the carbon atom to which R₃ is attached is a chiral carbon atom which has the stereochemical configuration of (1,3)-R,S, (1,3)-R,R, (1,3)-S,S or(1,3)-S,R; and
n is 1 or 2.

2. A compound of claim 1 which is 5-benzyloxycarbonyl-1-ethoxycarbonyl-5-azaspiro[2,4]heptane, 5-benzyloxycarbonyl-5-azaspiro[2,4]heptane-1-carboxylic acid, 5-benzyloxycarbonyl-1-azidocarbonyl-5-azaspiro[2,4]heptane, 5-benzyloxycarbonyl-1-isocyanato-5-azaspiro[2,4]heptane, 1-amino-5-azaspiro[2,4]heptane, 5-benzyl-1-ethoxycarbonyl-5-azaspiro[2,4]heptane, bis-(1,5-ethoxycarbonyl)-5-azaspiro[2,4]heptane, 5-ethoxycarbonyl-5-azaspiro[2,4] heptane-1-carboxylic acid, 5-(t-butoxycarbonyl)-1-ethoxycarbonyl-5-azaspiro[2,4]heptane, 5-t-butoxycarbonyl-1-t-butoxycarbonylamino-5-azaspiro[2,4]heptane, 1-t-butoxycarbonyl methylamino-5-t-butoxycarbonyl-5-azaspiro[2,4]heptane, 1-methylamino-5-azaspiro[2,4]heptane, 1-methylamino-5-azaspiro[2,4]heptane hydrochloride, 5-benzyl-1-(ethoxycarbonyl)-5-azaspiro[2,5]octane, 5-benzyl-oxycarbonyl-1-ethyloxycarbonyl-5-azaspiro[2,4]octane, 5-benzyloxycarbonyl-5-azaspiro[2,5]octane-1-carboxylic acid, 1-amino-5-azaspiro[2,5]octane, 1-amino-5-azaspiro[2,5]octanehydrochloride, 5-benzyl-1-benzylamido-5-azaspiro[2,4]heptane, 1-aminomethyl-5-azaspiro[2,4]heptane, 1-aminomethyl-5-azaspiro[2,4]heptaneformate, 1-hydroxymethyl-5-benzyl-5-azaspiro[2,4]heptane, 1-hydroxymethyl-5-azaspiro[2,4]heptane, 1-hydroxymethyl-5-azaspiro[2,4]heptane formate, 1-amino-5-ethoxycarbonyl-5-azaspiro[2,4]heptane, 1-hydroxy-5-azaspiro[2,4]heptane hydrochloride, 1-acetoxymethyl-5-t-butoxycarbonyl-5-azaspiro[2,4] heptane, 5-benzyl-1-ethoxycarbonyl-4-methyl-5-azaspiro[2,4]heptane, 5-benzyl-1-ethoxycarbonyl-6-methyl-5-azaspiro[2,4]heptane, 5-ethoxycarbony-1-ethoxycarbonyl-4-methyl-5-azaspiro[2,4]heptane, 5-ethoxycarbonyl-1-ethoxycarbonyl-6-methyl-5-azaspiro[2,4]heptane, 5-ethoxycarbonyl-4-methyl-5-azaspiro[2,4]heptane-1-carboxylicadid, 5-ethoxycarbonyl-6-methyl-5-azaspiro[2,4]heptane-1-carboxylic acdi, 5-ethoxycarbonyl-6-methyl-1-azidocarbonyl-5-azaspiro[2,4]heptane, 5-ethoxycarbonyl-4-methyl-1-azidocarbonyl-5-azaspiro-[2,4]heptane, 5-ethoxycarbonyl-1-azidocarbonyl-5-azaspiro[2,4]heptane, 5-ethoxycarbonyl-1-isocyanato-5-azaspiro[2,4]heptane, 5-ethoxycarbonyl-isocyanato-4-methyl-5-azaspiro[2,4]heptane, 5-ethoxycarbonyl-1-isocyanato-6-methyl-5-azaspiro[2,4]heptane, 1-amino-4-methyl-5-azaspiro[2,4]heptane, 1-amino-4-methyl-5-azaspiro[2,4]heptane hydrochloride, 1-amino-6-methyl-5-azaspiro[2,4]heptane, 1-amino-6-methyl-5-azaspiro[2,4]heptane hydrochloride, N-[1-(R)-phenylethyl]-5-(t-butoxycarbonyl)-5-azaspiro [2,4]heptane-1-carboxamide, (1S,3S)-N-[1-(R)-phenylethyl]-5-(t-butoxycarbonyl)-5-azaspiro[2,4]heptane-1-carboxamide, (1R,3R)-N-[1-(R)-phenylethyl]-5-(t-butoxycarbonyl)-5-azaspiro[2,4]heptane-1-carboxamide, (1R,3S)-N-[1-(R)-phenylethyl]-5-(t-butoxycarbonyl)-5-azaspiro[2,4]heptane-1-carboxamide, (1S,3R)-N-[1-(R)-phenylethyl]-5-(t-butoxycarbonyl)-5-azaspiro[2,4]heptane-1-carboxamide, (1R,3R)-5-azaspiro[2,4]heptane-1-carboxylic acid, (1S,3S)-5-azaspiro[2,4] heptane-1-carboxylic acid, (1S,3R)-5-azaspiro[2,4]heptane-1-carboxylic acid, (1R,3S)-5-azaspiro[2,4]heptane-1-carboxylic acid, (1S,3S)-5-(t-butoxycarbonyl)-5-azaspiro[2,4]heptane-1-carboxylic acid, (1R,3R)-5-(t-butoxycarbonyl)-5-azaspiro[2,4]heptane-1-carboxylic acid, (1S,3R)-5-(t-butoxycarbonyl)-5-azaspiro[2,4] heptane-1-carboxylic acid, (1R,3S)-5-(t-butoxycarbonyl)-5-azaspiro [2,4]heptane-1-carboxylic acid, (1S,3R)-1-amino-5-azaspiro[2,4] heptane, (1S,3R)-1-amino-5-azaspiro[2,4]heptane hydrochloride, (1S,3S)-1-amino-5-azaspiro[2,4]heptane, (1R,3R)-1-amino-5-azaspiro [2,4]heptane, (1S,3S)-1-amino-5-azaspiro[2,4]heptane hydrochloride, (1R,3S)-1-amino-5-azaspiro[2,4]heptane, (1R,3S)-1-amino-5-azaspiro [2,4]heptane hydrochloride, 1-methanesulfonyloxymethyl-5-benzyl-5-azaspiro[2,4]heptane and 5-benzyl-1-(p-toluenesulfonyloxymethyl)-5-azaspiro[2,4]heptane.

3. A compound of claim 1 which is:
1-amino-5-azaspiro[2,4]heptane, (1S,3S)-1-amino-5-azaspiro[2,4]heptane, (1S,3R)-1-amino-5azaspiro[2,4]heptane, (1R,3S)-1-amino-5-azasprio[2,4] heptane, (1R,3R)-1-amino-5-azaspiro[2,4]heptane or 1-aminomethyl-5-azaspiro[2,4]heptane or, 1-amino-5-azaspiro[2,4]octane and 1-methylamino-5-azaspiro[2,4]heptane or their hydrochloride salt.
